(19) Europäisches Patentamt
European Patent Office
Office européen des brevets

(11) Veröffentlichungsnummer: **0 628 568 A2**

# (12) EUROPÄISCHE PATENTANMELDUNG

(21) Anmeldenummer: **94108442.8**

(22) Anmeldetag: **01.06.94**

(51) Int. Cl.$^5$: **C07H 21/00**, C07H 19/10, C07H 19/20, C12Q 1/68, C12P 19/34, //C12Q1/70

(30) Priorität: **09.06.93 DE 4319151**
**16.11.93 DE 4339086**
**28.12.93 DE 4344742**

(43) Veröffentlichungstag der Anmeldung:
**14.12.94 Patentblatt 94/50**

(84) Benannte Vertragsstaaten:
**AT BE CH DE DK ES FR GB IT LI NL SE**

(71) Anmelder: **BOEHRINGER MANNHEIM GMBH**

**D-68298 Mannheim (DE)**

(72) Erfinder: **Kruse-Müller, Cornelia, Dr.**
**Oldenburger Strasse 3**
**D-26188 Edewecht (DE)**
Erfinder: **Berner, Sibylle, Dr.**
**Rettenberger Weg 21**
**D-82319 Starnberg (DE)**
Erfinder: **Kaletta, Cortina, Dr.**
**Seehauser Strasse 14**
**D-81379 München (DE)**

## (54) Verfahren zur Immobilisierung von Nukleinsäuren.

(57) Verfahren zur Immobilisierung von Nukleinsäuren durch Hybridisierung der Nukleinsäure mit einer Fangsonde, wobei die Fangsonde gegen enzymatische Verlängerung oder/und enzymatischen Abbau des gebildeten Hybrids geschützt ist.

EP 0 628 568 A2

Gegenstand der Erfindung sind Verfahren zur Immobilisierung von Nukleinsäuren, Verfahren zum Nachweis von Nukleinsäuren unter Einbeziehung dieser Immobilisierung, Reagenzien zur Durchführung dieses Verfahrens und entsprechende Reagenzkits.

Beim Nachweis von Nukleinsäuren unterscheidet man zwischen homogenen und heterogenen Verfahrensführungen. Während bei homogenen Verfahren Nachweissonden und nachzuweisende Nukleinsäure während der Messung in Lösung vorliegen, laufen heterogene Verfahren unter Immobilisierung der nachzuweisenden Nukleinsäure ab. Dies kann direkt unter kovalenter Bindung der Nukleinsäure an eine feste Phase oder über Kopplung der Nukleinsäure an einen Partner eines Affinitätspaares und Bindung des anderen Partners des Affinitätspaares an eine feste Phase geschehen.

Eine Kopplungsmöglichkeit besteht auch durch Immobilisierung der Nukleinsäure durch Hybridisierung mit einer festphasengebundenen, zumindest teilweise zu der nachzuweisenden Nukleinsäure komplementären sogenannten Fangsonde. Die Fangsonde kann auf beliebige Weise an eine feste Phase gebunden sein oder werden. Vorteil des heterogenen Verfahrens ist die einfache Möglichkeit der Abtrennung markierter Reagenzbestandteile von den nachzuweisenden Nukleinsäuren.

Beispiele für Verfahren, bei denen nachzuweisende Nukleinsäuren über Fangsonden immobilisiert werden, sind für direkte Bindung in EP-A-0 079 139 und für Bindung über ein Affinitätspaar in EP-A-0 192 168 beschrieben. Den dort beschriebenen Verfahrensführungen ist charakteristisch, daß die nachzuweisenden Nukleinsäuren mit einer Nachweissonde, die ebenfalls mit der nachzuweisenden Nukleinsäure hybridisieren kann, hybridisiert werden und das gebildete Hybrid nach Entfernung überschüssiger Nachweissonde an der festen Phase über eine Markierungsgruppe der Nachweissonde nachgewiesen wird.

Durch den Einzug von targetspezifischen Amplifikationsverfahren in die Nukleinsäurediagnostik wurde es möglich, aus üblicherweise selten vorkommenden Nukleinsäuren eine große Menge von Nukleinsäuren herzustellen. Ein solches Verfahren, bei dem auch eine Digoxigenin-Markierungsgruppe in die amplifizierte Nukleinsäure eingebaut wird, ist in der WO 92/06216 beschrieben.

Aus PCT/US 92/01735 ist ein Verfahren zur Herstellung von 2'-substituierten Nukleinsäuren und deren Resistenz gegen Abbau durch Restriktionsenzyme bekannt. Es ist ferner bekannt, daß 3'-Deoxynukleotide bei der Sequenzierung von Nukleinsäuren als Kettenabbruchreagenzien und zur Verhinderung der Verlängerung von Nukleinsäuren in 3'-Richtung wirken.

Aufgabe der Erfindung war es, ein besonders einfaches und effizientes Immobilisierungsverfahren zur Verfügung zu stellen.

Gegenstand der Erfindung ist ein Verfahren zur Immobilisierung von Nukleinsäuren durch Hybridisierung der Nukleinsäure mit einer Fangsonde, wobei die Fangsonde gegen enzymatische Verlängerung oder/und enzymatischen Abbau des gebildeten DNA-/RNA-Hybrids geschützt ist.

Weitere Gegenstände sind ein Verfahren zum Nachweis einer Nukleinsäure unter Immobilisierung nach diesem Verfahren, sowie dafür geeignete Reagenzien.

Eine Fangsonde ist eine Nukleinsäure, die mit einer anderen Nukleinsäure hybridisieren kann und die entweder immobilisiert oder immobilisierbar ist. Immobilisierung ist ein Vorgang der Bindung einer Nukleinsäure an eine feste Phase. Sie kann entweder direkt (kovalente Bindung der Fangsonde an die Oberfläche der festen Phase) oder indirekt (über ionische, adsorptive oder biospezifische Bindung) geschehen. Bevorzugte Fangsonden im Sinne der Erfindung sind immobilisierbar mit einem Partner eines biospezifischen Paares markierte Nukleinsäuren. Biospezifische Paare sind die Paare Antigen/Antikörper, Hapten/Antikörper, Vitamin/Rezeptor, Hormone/Rezeptor und Zucker/Lektin. Bevorzugt sind die Paare Biotin/Avidin, Biotin/Streptavidin und Hapten/Antikörper. Solchermaßen markierte Nukleinsäuren können z. B. durch chemische Oligonukleotidsynthese mittels entsprechend markierten aktiven Mononukleosidderivaten oder durch enzymatische Synthese über Einbau entsprechend markierter Mononukleosidtriphosphate oder chemische Modifizierung schon fertig synthetisierter Nukleinsäuren hergestellt werden.

Enzymatische Verlängerung ist ein Vorgang, der in lebenden Zellen oder davon abgeleiteten Flüssigkeiten, die noch aktive Enzyme enthalten, abläuft. Ursache sind Nukleinsäuren-verlängernde Enzyme, wie z. B. Polymerasen. Diese Verlängerungsreaktionen können die Bestimmung von Nukleinsäuren in erheblichem Maß stören, insbesondere, wenn der Probe zur Durchführung einer Amplifikation entsprechend Enzyme zugesetzt wurden.

Zur Unterdrückung dieser Verlängerungsreaktion wird erfindungsgemäß die Fangsonde gegen Verlängerung geschützt. Dies kann geschehen durch Anbringen von chemischen Resten, die den sterischen Zutritt der Verlängerungsenzyme an das 3' oder 5'-Ende verhindern. Da diese Reste manchmal die Hybridisierung der Fangsonde mit der zu immobilisierenden Nukleinsäure behindern oder sogar unmöglich machen, ist es bevorzugt, die Hydroxylgruppe, an der die Verlängerung stattfinden würde, durch einen inaktiven Rest zu ersetzen.

Solche Reste sind z. B. Wasserstoff oder $C_1$-$C_3$-Alkylgruppen, bevorzugt ist Wasserstoff. Die damit entstandenen Fangsonden enthalten daher am Ende einen 3'- oder 5'-Desoxyribonukleosidrest oder 2', 3'- oder 2', 5'-Dideoxy-ribonukleosidrest. Bevorzugt ist das 3'-Ende der Fangsonde geschützt.

Enzymatischer Abbau ist ebenfalls ein weitverbreitetes Phänomen in biologischen Probenflüssigkeiten. Ursache sind Endo-, oder Exonukleasen, wie DNAsen und RNAsen. Ebenfalls eingeschlossen sind Restriktionsenzyme. Wenn beispielsweise RNA als zu immobilisierende Nukleinsäure verwendet werden soll, so würde diese RNA durch RNAseH abgebaut, sobald ein Hybrid aus DNA als Fangsonde und der RNA gebildet wird. Das bedeutet, daß ein Immobilisierungsverfahren mittels unmodifizierter DNA-Fangsonden sehr ineffizient oder gar unmöglich wäre. Die vorliegende Erfindung bekämpft die Ursache durch Modifizierung der Fangsonde, so daß die genannten Enzyme das Hybrid aus der Fangsonde und nachzuweisender Nukleinsäure nicht mehr als Substrat erkennen.

Als recht wirksam hat es sich erwiesen, Zuckerreste der Fangsonde zu modifizieren, z. B. in 2'-Position. Als bevorzugt haben sich für die Immobilisierung von RNA in Anwesenheit von RNAseH die Verbindungen erwiesen, bei denen die 2'-Hydroxylgruppe von Desoxyribonukleotiden durch O-Alkyl oder O-Alkenylgruppen ersetzt ist. Besonders bevorzugt sind die in WO 91/15499 beschriebenen Verbindungen.

Erstaunlicherweise können Nukleinsäuren durch gleichzeitige Vornahme der beschriebenen Maßnahmen effektiv gegen enzymatischen Abbau des RNA/DNA-Hybrids und enzymatische Verlängerung geschützt werden. Die Modifizierungen sind so gewählt, daß sich ihre Wirkungen gegenseitig nicht nachteilig beeinflussen.

Als Fangsonde eignen sich Oligo- oder Polynukleotide, die auch weiter modifiziert sein können, jedoch nicht in einer Weise, daß eine Hybridisierung durch Basenpaarung mit der zu immobilisierenden Nukleinsäure unmöglich gemacht wird.

Immobilisierte Fangsonden sind Nukleinsäuren, die an eine feste Phase gebunden sind. Die Bindung kann kovalent durch Ausbildung chemischer Bindungen oder biospezifisch über zwei Partner einer biospezifischen Bindung (z. B. Hapten/Antikörper, Vitamin/Rezeptor, bevorzugt Biotin/Streptavidin) geschehen, wobei einer der Partner an die Fangsonde gebunden ist, der andere hingegen an die feste Phase.

Immobilisierbare Fangsonden sind Nukleinsäuren, die noch nicht an eine feste Phase gebunden sind, jedoch in oben genannte immobilisierte Fangsonden überführt werden können. Dazu enthalten die immobilisierbaren Fangsonden entweder einen chemisch mit der Festphase reaktiven Rest (z. B. photoaktivierbare Reste) oder einen Partner einer biospezifischen Bindung.

Als feste Phase kommen beispielsweise Küvetten, Mikrotiterplatten oder Partikel (z. B. plastikbeschichtete Magnetpartikel) in Frage.

Zur erfindungsgemäßen Immobilisierung der Nukleinsäuren wird die Fangsonde mit einer Probe, welche die zu immobilisierenden Nukleinsäuren enthält, in Kontakt gebracht. Die Hybridisierung findet unter Bedingungen statt, die von der Länge der beteiligten Nukleinsäure und der Fangsonde abhängen. Der Fachmann ist in der Lage, diese Bedingungen durch wenige Versuche, z. B. durch Ermittlung der Schmelztemperatur, zu ermitteln. Sie werden sich jedoch im allgemeinen nicht von den Bedingungen unterscheiden, die für Nukleinsäuren der gleichen (unmodifizierten) Nukleotidsequenz wie die der Fangsonde gelten.

Sofern es sich bei der Fangsonde um eine bereits immobilisierte Nukleinsäure handelt, findet gleichzeitig mit der Hybridisierung der Fangsonde mit der Nukleinsäure die Immobilisierung der Nukleinsäure statt. Falls gewünscht, kann nun das gebildete Hybrid mit der Festphase von der Flüssigkeit, in der die Nukleinsäure enthalten war, abgetrennt werden. Das festphasengebundene Hybrid kann anschließend gewaschen werden und steht gegebenenfalls für weitere Reaktionen zur Verfügung.

Handelt es sich bei der Fangsonde um eine immobilisierbare Fangsonde, so wird die das Hybrid enthaltende Flüssigkeit mit einer Festphase in Kontakt gebracht, welche die Fangsonde immobilisieren kann. Die feste Phase enthält dazu bevorzugt einen Bindungspartner der Gruppe, mit der die Fangsonde modifiziert ist. Wie bei immobilisierten Fangsonden können anschließende Prozesschritte durchgeführt werden.

Die zu immobilisierende Nukleinsäure kann beliebiger Art und Ursprungs sein. Es kann sich um Nukleinsäuren handeln, wie sie in natürlichen Proben, z. B. Körperflüssigkeiten oder Kulturproben, aber auch in durch Prozesschritte aus natürlichen Proben hergestellten Proben vorkommen. Sie können daher modifiziert oder unmodifiziert sein. Die Modifikationen können sich auf die Sequenz, die Länge, oder/und auch die chemische Struktur der Nukleinsäuren beziehen. Die Nukleinsäuren sind in einem bevorzugten Fall Kopien einer ursprünglich in der Probe enthaltenden Nukleinsäure oder Nukleinsäuresequenz. Bei den Kopien handelt es sich weiter bevorzugt um Amplifikate, die in einer in-vitro Vermehrungsreaktion eines Teils einer ursprünglichen Nukleinsaure entstanden sind. Besonders bevorzugt sind Amplifikate, die durch enzymatische Verlängerung von Nukleinsäuren an der ursprünglichen Nukleinsäure als Templat gebildet

wurden. Beispiele für solche in-vitro Vermehrungsreaktionen sind die PCR, die LCR oder promotergesteuerte Amplifikationen.

In einer weiteren bevorzugten Ausführung sind die Nukleinsäuren durch Einbau detektierbarer Gruppen modifiziert. Dieser Einbau kann duch Enzymreaktion oder chemische Reaktion geschehen. Besonders bevorzugt ist die Enzymreaktion, wie sie bei der Amplifikation wie oben geschildert vorkommt. In diesem Fall hat es sich als besonders zweckmäßig herausgestellt, detektierbar modifizierte Mononukleosidtriphosphate zur Verlängerung einzusetzen. Bevorzugt werden dann nicht eingebaute detektierbare Reste abgetrennt. Ist die zu immobilisierende Nukleinsäure detektierbar modifiziert, ist sie natürlich im immobilisierten Zustand an der festen Phase auf bekannte Weise nachweisbar. Ein Nachweisschritt kann sich daher an das Immobilisierungsverfahren anschließen. Ebenfalls Gegenstand der Erfindung ist ein Verfahren zum Nachweis von Nukleinsäuren unter Verwendung des erfindungsgemäßen Immobilisierungsverfahrens mit anschließendem Nachweis der immobilisierten Hybride. Besonders bevorzugt findet der Nachweis durch Bestimmung von detektierbaren Gruppen an der immobilisierten Nukleinsäure satt.

Detektierbare Gruppen sind direkt oder indirekt nachweisbare Gruppen. Direkt nachweisbare Gruppen sind beispielsweise radioaktive ($^{32}P$), farbige, oder fluoreszierende Gruppen oder Metallatome. Indirekt nachweisbare Gruppen sind beispielsweise immunologisch oder enzymatisch wirksame Verbindungen, wie Antikörper, Antigene, Haptene oder Enzyme oder enzymatisch aktive Teilenzyme. Diese werden in einer nachfolgenden Reaktion oder Reaktionssequenz detektiert. Besonders bevorzugt sind Haptene, da mit ihnen markierte Nukleosidtriphosphate im allgemeinen besonders gut als Substrate von Polymerasen einsetzbar sind und eine anschließende Reaktion mit einem markierten Antikörper gegen das Hapten oder das haptenisierte Nukleosid leicht vorgenommen werden kann. Solche Nukleosidtriphosphate sind beispielsweise Brom-Nukleosidtriphosphate oder Digoxigenin, Digoxin- oder Fluorescein-gekoppelte Nukleosidtriphosphate. Als besonders geeignet haben sich die in EP-A-0 324 474 genannten Steroide und deren Detektion erwiesen. Für deren Inkorporation in Nukleinsäuren wird hiermit auf die EP-A-0 324 474 verwiesen.

In einer ersten Ausführungsform des erfindungsgemäßen Verfahrens wird die nachzuweisende Nukleinsäure über eine an sie anhybridisierte Nachweissonde nachgewiesen. Diese Nachweissonde hat eine Nukleotidsequenz, die zu einem anderen Teil der nachzuweisenden Nukleinsäure hybridisieren kann als die Fangsonde. Es bildet sich daher ein sogenanntes Sandwichhybrid aus Fangsonde, nachzuweisender Nukleinsäure und Nachweissonde aus. Die Reihenfolge der Hybridisierung dieser drei Nukleinsäuren ist prinzipiell beliebig. Bevorzugt findet die Sandwichbildung im wesentlichen gleichzeitig statt. In einer besonders bevorzugten Ausführungsform des Sandwichtests wird die Fangsonde der Probe mit der nachzuweisenden Nukleinsäure zugesetzt, dann die nachzuweisende Nukleinsäure oder Teile davon amplifiziert und anschließend die unter Hybridisierungsbedingungen gebildeten Hybride unter Verwendung einer Nachweissonde nachgewiesen. Dabei ist es möglich, auch die Nachweissonde entsprechend der Fangsonde gegen enzymatische Verlängerung/Abbau zu schützen und ebenfalls vor oder während der Amplifikation zuzusetzen.

In einer bevorzugten Ausführungsform wird die nachzuweisende Nukleinsäure in Anwesenheit einer an einer festen Phase immobilisierten Fangsonde unter enzymatischem Einbau von detektierbar markierten Nukleotiden, besonders Mononukleotiden, amplifiziert. Nach eventueller Abtrennung überschüssiger Mononukleotide wird die an der festen Phase immobilisierten Nukleinsäure über die Menge an eingebauten detektierbaren Nukleotiden bestimmt. Als besonders bevorzugt als Amplifikation hat sich erfindungsgemäß das in EP-A-0 329 822 beschriebene Verfahren erwiesen. Es ist hier von besonderem Vorteil, wenn die Fangsonde so modifiziert ist, daß sie die zu immobilisierende RNA (Transkripte) vor Abbau aus dem gebildeten Hybrid aus Fangsonde und RNA schützt.

Bei Verwendung einer immobilisierbaren Fangsonde gibt es zwei Ausführungsformen. In einer ersten wird die nachzuweisende Nukleinsäure in Anwesenheit der immobilisierbaren Fangsonde und detektierbar markierten Mononukleotiden amplifiziert. Alle Amplifikationsverfahren können verwendet werden. Bevorzugt ist die Polymerase-Ketten-Reaktion
(EP-B-0 201 184) mit Thermocyclen zur Denaturierung zwischen den Cyclen; die Amplifikation kann in einem beliebigen Gefäß durchgeführt werden. Anschließend wird das Reaktionsgemisch in ein Gefäß überführt, an dessen feste Phase die immobilisierbare Fangsonde immobilisiert werden kann. Überschüssige Mononukleotide werden bevorzugt abgetrennt und die Markierung an der festen Phase als Maß für die Anwesenheit der nachzuweisenden Nukleinsäure detektiert.

Bei einer zweiten, bevorzugten Ausführungsform, findet die Amplifikation bereits in einem Gefäß statt, an dessen Innenwand die immobilisierbare Nukleinsäure gebunden werden kann. Dies hat den Vorteil, daß ein Umfüllen des Amplifikationsansatzes in ein weiteres Gefäß entfallen kann. Im Fall der Durchführung von thermocyclisch geführten Amplifikationen, z.B. der PCR oder LCR, ist dazu erfindungsgemäß die Verwendung thermostabiler Gefäße und Beschichtungen erforderlich.

Sofern das Reaktionsgefäß schon zur Detektion der Hybride geeignet ist und Direktmarkierungen eingesetzt werden, kann die Reaktionsführung vom Start der Amplifikation bis zum Ende der Detektion ohne Zugabe von Reagenzien erfolgen.

Während die bisher bekannten Verfahren unter dem Nachteil litten, daß die Reaktionsmischung nach der Amplifikation zur Zugabe der Sonden der Umgebung ausgesetzt werden mußte (Kontaminationsprobleme), so hat die Zugabe der Sonden schon mit den Reagenzien zur Amplifikation den Vorteil, daß erstens weniger Pipettierschritte erforderlich sind und zweitens ein Öffnen des Reaktionsgefäßes nach der Amplifikation und Zugabe von Reagenzien (z.B. der Fangsonde) entfallen kann. Besonders vorteilhaft wirkt sich diese Charakteristisk für Verfahren mit Direktmarkierungen, die zur Detektion der Markierung nicht die Zugabe von Reagenzien erfordern, aus. Für diesen Fall, z. B. Elektrochemilumineszenzmarkierungen, muß vom Start der Amplifikation bis zur Messung des analytabhängigen Signals kein Reagenz zugegeben werden. Die Zugabe von Reagenzien ist jedoch jederzeit möglich.

Ein weiterer Gegenstand der Erfindung sind Nukleinsäuren, die sowohl gegen enzymatischen Abbau von Nukleinsäuren in Hybriden aus der Nukleinsäure und komplementären Nukleinsäuren modifiziert sind, als auch vor enzymatischen Verlängerung geschützt sind und deren Verwendung als Fangsonden zur Immobilisierung von Nukleinsäuren. Bevorzugt sind diese Nukleinsäuren an den zur Wasserstoffbrückenbindung zu komplementären Nukleinsäuren fähigen Positionen der Nukleobase, insbesondere an den Aminofunktionen, nicht gegenüber den natürlichen Nukleobasen verändert.

Ebenfalls Gegenstand der Erfindung ist ein Reagenzkit enthaltend einen ersten Behälter A mit einem Enzym zur Verlängerung von Nukleinsäuren und einen zweiten Behälter B mit einer erfindungsgemäßen Fangsonde.

In diesen oder weiteren Behältern sind bevorzugt alle ferner übrigen, zur Immobilisierung bzw. Amplifikation oder Detektion von Nukleinsäuren erforderlichen Reagenzien, wie Puffer, Mononukleosidtriphosphate, Markierungsreagenzien etc.) enthalten.

Besonders bevorzugt enthält Behälter B ferner entweder eine detektierbare Nachweissonde, die ebenfalls erfindungsgemäß modifiziert ist, oder detektierbare Mononukleosidtriphosphate. Außerdem kann Behälter A ein Nukleinsäuren, insbesondere ein RNA in einem RNA/DNA-Hybrid, abbauendes Enzym beinhalten.

Gegenstand der Erfindung sind außerdem spezielle elektrochemilumineszenzmarkierte Mononukleosidtriphosphate.

Reste, welche elektrochemilumineszente Eigenschaften haben, sind bekannt. Sie werden im folgenden Elektrochemilumineszenzgruppe E genannt. Besonders bewährt als elektrochemilumineszente Bestandteile von E haben sich Koordinationskomplexe K mit einem Atomgewicht von mehr als 500 g/mol, bevorzugt 550 bis 2000 g/mol. Als Metallionen sind bevorzugt Ionen der Nebengruppen VII und VIII des Periodensystems, besonders bevorzugt Ru, Os, Re, ganz besonders bevorzugt ist Ruthenium.

Als Liganden des Metallions in dem Koordinationskomplex haben sich insbesondere organische Liganden bzw. Reste bewährt, wie z. B. in WO 92/10267 oder EP-A-0 340 605 beschrieben.

Als organische Liganden sind Kohlenwasserstoffe geeignet, die Atome enthalten, die Elektronendonoren für das entsprechende Metallatom sind, z. B. Stickstoff, Sauerstoff oder Schwefel. Die Elektronendonoren sind in geeigneter Weise geometrisch angeordnet, so daß eine Komplexbildung mit dem Metallion möglich ist. Im Falle von Ruthenium sind Bipyridylreste als Liganden bevorzugt.

Einer der Liganden hat außerdem eine Valenz, über die der Komplex an ein Mononukleosidtriphosphat gebunden ist. Bevorzugt befindet sich zwischen dem Koordinationskomplex und den Atomen des Nukleosidtriphosphats eine Abstandsgruppe A. Die Abstandsgruppe besteht bevorzugt aus einer mindestens 3, bevorzugt 5 bis 15, besonders bevorzugt 4 bis 10 Atomen langen Atomkette, an welche weitere Reste seitlich gebunden sein können. Die Atomkette enthält bevorzugt eine Kohlenwasserstoffkette, die durch Heteroatome, z. B. -O-(Sauerstoff) oder die Gruppe-$NR^2$-, bei der N Stickstoff und $R^2$ Wasserstoff oder ein $C_1$-$C_6$-Alkylrest ist, unterbrochen sein kann. Bevorzugt enthält die Atomkette mindestens einmal den Bauteil -($CR^3_2$-$CR^3_2$-0)-, in dem $R^3$ Wasserstoff oder ein $C_1$-$C_6$-Alkylrest ist. Die Abstandsgruppe hat bevorzugt ein Molekulargewicht von weniger als 1000 g/mol.

Die Elektrochemilumineszenzgruppe E ist bevorzugt kovalent an ein Atom der Nukleobase N des Mononukleotids gebunden.

Bevorzugte Verknüpfungsstellen sind C5, C6, C4 bzw. N4 bei Pyrimidinen und C8 bzw. N6 bei Purinen und C7 bei Deazapurinen.

Bevorzugt sind Mononukleosidtriphosphate der Formel I

P-Z-B-E (I),

in der

P ein Triphosphatrest oder triphosphatanaloger Rest,
Z ein Zucker oder zuckeranaloger Rest,
B eine Nukleobase oder nukleobasenanaloger Rest und
E eine Elektrochemilumineszenzgruppe

bedeuten.

Triphosphat, Zucker und Nukleobase sind die in natürlich vorkommenden Mononukleosidtriphosphaten enthaltenen Reste. Dazu analoge Reste sind Reste, die durch Ersatz eines oder mehrerer Atome dieser Reste erhältlichen Reste, die dennoch die Eigenschaft der Verbindungen der Formel I, in Nukleinsäuren eingebaut werden zu können, nicht wesentlich behindern.

Triphosphatanaloge sind z. B. Reste, bei denen einer oder mehrere der Sauerstoffatome durch Schwefelatome ersetzt sind. Zuckeranaloge sind z. B. Reste, bei denen das endocyclische Sauerstoffatom durch die Gruppe $-CR^4{}_2-$ ersetzt ist, in der $R^4$ Wasserstoff oder eine $C_1$-$C_3$-Alkylgruppe ist. Nukleobasenanaloge sind beispielsweise Deazapurine, bevorzugt 7-Deazapurine.

Bevorzugte Mononukleosidtriphosphate sind Verbindungen der Formel Ia

Ia

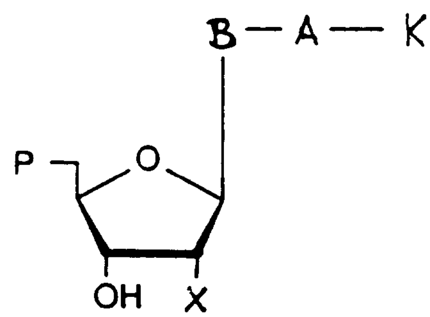

wobei

X Wasserstoff oder die Gruppe $OR^1$,
$R^1$ ein $C_1$-$C_6$ -Alkylrest, $C_1$-$C_6$ -Alkylenrest oder Wasserstoff
B ein Purin-, Deazapurin- oder Pyrimidinrest ist
A eine Abstandsgruppe und
K ein elektrochemilumineszenter Koordinationskomplex

ist

sowie deren Alkali- oder Erdalkalisalze.

Überraschenderweise hat es sich gezeigt, daß selbst so große Reste wie die beschriebenen Metallkomplexe den Einbau von damit markierten Mononukleosidtriphosphaten durch Enzyme, die den Einbau von Mononukleosidtriphosphaten in Nukleinsäuren katalysieren, nicht wesentlich stören. Die eingebaute Menge an markierten Mononukleosidtriphosphaten ist für den Nachweis damit markierter Nukleinsäuren über Elektrochemilumineszenz ausreichend und verglichen mit den Verfahren des Standes der Technik, die mit Elektrochemilumineszenz arbeiten, ist das erfindungsgemäße Verfahren sensitiver.

Besonders überraschend ist, daß der dynamische Meßbereich, d. h. das Verhältnis der bei einer bestimmten vorgegebenen Mononukleosidtriphosphatkonzentration obersten quantifizierbaren Menge an zu bestimmenden Nukleinsäuren zu der untersten quantifizierbaren Menge sehr hoch ist. Er kann bis über $10^7$ liegen.

Der Einbau der markierten Mononukleosidtriphosphate kann auf verschiedene Weise geschehen.

Eine Möglichkeit ist die Verlängerung von an die nachzuweisende Nukleinsäure anhybridisierten Oligonukleotiden, sogenannten Primern. Es wird unter Katalyse durch eine Polymerase, bevorzugt DNA-Polymerase, an das 3'-Ende des Primers ein Nukleinsäurestück angehängt, welches im wesentlichen komplementär zu dem entsprechenden Stück der nachzuweisenden Nukleinsäure ist. Dieses Verfahren kann unter Verwendung der gebildeten Verlängerungsprodukte cyclisch unter Amplifikation eines Teilstükkes der anwesenden Nukleinsäuren ablaufen. Ein solches Verfahren ist beispielsweise beschrieben in EP-A-0 201 184.

Eine weitere Möglichkeit ist die Replikation oder Transkription von Nukleinsäuren, welche ein Origin of replication oder einen Promoter enthalten. In diesem Fall werden die Nukleinsäuren nur aus Mononukleotiden aufgebaut, ohne Verlängerung eines Primers. Beispiele sind aus der WO 91/03573 oder WO 91/02818 bekannt.

Für die Durchführung der Einbaureaktionen wird in der Reaktionsmischung mindestens eine Sorte von Mononukleotiden ((d)ATP, (d)CTP, (d)GTP oder dTTP/(d)UTP) teilweise oder ganz durch das elektrochemilumineszenzmarkierte Mononukleosidtriphosphat ersetzt. Bevorzugt wird eine Mononukleosidtriphosphatart (z. B. dUTP) zu 10 bis 50 % durch das markierte Analog ersetzt (z. B. rutheniumkomplexmarkiertes dUTP). Die Konzentration des markierten Mononukleosidtriphosphats liegt bevorzugt bei 20 bis 70 µM, besonders bevorzugt bei 25 bis 40 µM. Die übrigen Reaktionsbedingungen für den Einbau unterscheiden sich nicht wesentlich von den in den genannten Dokumenten beschriebenen Reaktionsbedingungen.

Die elektrochemilumineszenzmarkierten Mononukleosidtriphosphate können, je nach Struktur, auf verschiedene Weise hergestellt werden.

Soll die Gruppe an der Nukleobase angebracht sein, so kann eine Aminogruppe der natürlichen Basen mit einem elektrophilen Rest, z. B. einer aktivierten Ester-Gruppe der Abstandsgruppe umgesetzt werden.

Als besonders praktikabel hat es sich jedoch erwiesen, Mononukleosidtriphosphate, die bereits an einem Atom der Base eine Abstandsgruppe mit einer Aminofunktion aufweisen, mit einer solchen reaktiven Elektrochemilumineszenzverbindung umzusetzen. Ein Beispiel hierfür sind in 5-Position mit einer Alkylaminogruppe substituierte Pyrimidine enthaltende Mononukleosidtriphosphate (Proc. Acad. Sci. USA 78, 6633 - 37 (1991).

Mit einer reaktiven Gruppe versehene elektrochemilumineszente Verbindungen sind beispielsweise von der Firma IGEN erhältlich. Die Bildung eines Amids verläuft im wesentlichen unter Bedingungen, die zu normalen Amidbildungen aus aktivierten Estern und Aminen analog sind.

In Fig. 1 ist ein Schema eines erfindungsgemäßen Nachweisverfahrens unter Verwendung der PCR als Amplifikationsverfahren gezeigt.

In Fig. 2 ist das erfindungsgemäße Verfahren am Beispiel der NASBA-Amplifikation gezeigt.

In FIG. 3 ist das Ergebnis von Bestimmungen von Hpbadw21-DNA gemäß Erfindung (PCR mit Sonde) mit PCR ohne Sonde (dunkle Balken) verglichen.

In FIG. 4 ist dasselbe für eine Listeria-Bestimmung mittels NASBA dargestellt (mit Sonde: dunkle Balken).

In FIG. 5 ist das Ergebnis einer Bestimmung von HIV I mittels Elektrochemilumineszenzlabel in Anwesenheit der Fangsonde mit NASBA gezeigt.

In FIG. 6 ist ein Reaktionsschema zur Amplifikation und Detektion von Nukleinsäuren mittels NASBA gezeigt, wobei in die Transkripte ein Digoxigenin-markiertes Mononukleosid eingebaut wird.

In FIG. 7 ist die Struktur von $Ru(bpyr)_3$-AA-dUTP gezeigt.

In FIG. 8 ist die Struktur von $Ru(bpyr)_3$-DADOO-dUTP gezeigt.

In FIG. 9 ist ein Agarosegel gezeigt, bei dem die Unterschiede im Molekulargewicht der markierten Nukleinsäuren deutlich werden.

FIG. 10 zeigt den Nachweis von Amplifikaten durch Hybridisierung mit einer Rutheniummarkierten Sonde im Gel.

FIG. 11 zeigt den Einfluß des Denaturierungstyps auf die ECL-Intensitäten.

FIG. 12 zeigt die Abhängigkeit der Signalhöhe von der Menge an eingesetzten Rutheniummarkierten Mononukleotiden.

FIG. 13 zeigt den mengenabhängigen Nachweis von Nukleinsäuren beim Einbau von Rutheniumlabeln.

FIG. 14 zeigt einen Vergleich zwischen verschiedenen Ausführungsformen der Erfindung. Mit den senkrecht schraffierten Balken ist die Ausführungsform gemeint, bei der ein zweites Gefäß verwendet wird, welches die Fangsonde spezifisch binden kann, während die quer schraffierten Balken ein mit Amplifikation und Wandbindung in einem einzigen Gefäß arbeitendes Beispiel betreffen. Die gepunkteten Balken betreffen eine Ausführungsform, bei der das Reaktionsgemisch zur Detektion der bei der Enzymreaktion entwickelten Farbe in Lösung in ein Detektionsgefäß überführt wird.

Die folgenden Beispiele sollen die Erfindung näher erläutern:

Beispiel 1

HBV-Nachweis mit direktem und nachfolgendem Einsatz von Fangsonde

Als Target für die Polymerase-Ketten-Reaktion (EP-A-0 200 362) (PCR) wurde Hpbadw21 in den Konzentrationen von 1 ng bis 100 atg pro Ansatz verwendet. Das PCR-Ansatzvolumen betrug 100 µl.

Das Ansatzvolumen setzte sich wie folgt zusammen:

| Endkonzentration | |
|---|---|
| Primer 1 (SEQ. ID. NO. 1) | 200 nM |
| Primer 2 (SEQ. ID. NO. 2) | 200 nM |
| dATP (Boehringer Mannheim) | 200 µM |
| dCTP (Boehringer Mannheim) | 200 µM |
| dGTP (Boehringer Mannheim) | 200 µM |
| dTTP (Boehringer Mannheim) | 175 µM |
| dig-11-dUTP (Boehringer Mannheim GmbH, Best. Nr 1093088) | 25 µM |
| PCR-Puffer | 1 X |
| Taq-DNA-Polymerase (Perkin Elmer) | 2,5 U |
| PCR-Mix | 99 µl |
| Probe | 1 µl |
| Ansatzvolumen | 100 µl |

| | |
|---|---|
| 10 X PCR Puffer: | 100 mM Tris/HCl, 500 mM KCl, 15 mM $MgCl_2$, 1 mg/ml Gelatine, pH 9,0 |
| Primer 1: | Oligodesoxynukleotid, 18 mer, d (GGAGTGTGGATTCGCACT) (Pos. 2267-2284; EMBL, subtyp adw, SEQ. ID. NO. 1) |
| Primer 2: | Oligodesoxynukleotid, 18mer, d(TGAGATCTTCTGCGACGC) (Pos. 2436c-2419c, EMBL, Subtypadw, SEQ. ID. NO. 2) |
| Hpbadw 21: | klonierte HBV-DNA; Nukleotid 29 - 2606 (EMBL) der $HBV_{adw}$-Sequenz kloniert in pUCBM20 (Boehringer Mannheim) und linearisiert. |
| Biotinylierte Fangsonde: | d(AGCCTATAGACCACCAAATGCCCC TAT) 5'-biotinyliert (SEQ. ID. NO. 3) Pos. 2290-2316; EMBL, subtyp adw Lit.: Ono et al. Nucl. Acids Res. 11 (1983). |

Die PCR-Ansätze wurden in einem Perkin Elmer Thermal Cycler mit folgenden Cyclebedingungen amplifiziert: 3' 93 °C; (1' 94 °C, 1' 50 °C, 2' 70 °C) x 30; 5' 95 °C, 37 °C.

Die mit Biotin markierte Sonde wurde in einer Konzentration von 100 ng pro Ansatz eingesetzt. Für den Einsatz der Sonde während der PCR wurde sie mit Bio-16-ddUTP (Boehringer Mannheim, Best. Nr. 1427, 598) am 3' Ende blockiert (Lit.: Schmitz, G.G. et al. (1990) Anal. Biochem. 192, 222).

Die Detektion erfolgte auf mit Streptavidin beschichteten Mikrotiterplatten (Boehringer Mannheim GmbH, EP-A-0 269 092).

1. Nachweis der PCR ohne Sonde mit nachfolgender Hybridisierung:

Nach der Amplifikation wurden 20 µl einer PCR-Reaktion mit 40 ng Biotin markierter Fangsonde versetzt, für 5'auf 95 °C erhitzt, auf Eis gekühlt und für 15 min bei 37 °C inkubiert. Dazu wurden 180 µl Hybridisierungspuffer (50 mM Phosphatpuffer, 750 mM NaCl, 75 mM Na-Citrat, 0,05 % RSA, pH 6,8) gegeben, die Mischung in eine Kavität einer mit Streptavidin (SA) beschichteten Mikrotiterplatte pipettiert und 1 Stunde bei 37 °C inkubiert. Nach dreimaligem Waschen mit 0,9 % NaCl wurden 200 µl Konjugatpuffer 100 mM Tris x HCl, 0,9 % NaCl, 1 % RSA, pH 7,5 mit 0,2 U/ml Anti-Digoxigenin-POD (Boehringer Mannheim) hinzupipettiert und 20 min bei 37 °C inkubiert. Nach erneutem dreimaligem Waschen wurde mit 200 µl ABTS (1,9 mmol/l) detektiert und die Extinktion bei Hg 405 nm gemessen.

2. Nachweis der PCR mit direktem Einsatz der Sonde schon während der PCR (erfindungsgemäß):

Die Amplifikation wurde in Gegenwart von 100 ng Fangsonde durchgeführt.

Nach der Amplifikation wurden 20 µl einer PCR-Reaktion direkt mit 180 µl Hybridisierungspuffer versetzt und wie unter 1. beschrieben behandelt.

Die Ergebnisse sind in Fig. 3 dargestellt.

Beispiel 2

Promotergesteuerte Amplifikation mit und ohne direktem Einsatz einer Sonde (NASBA)

Als Analyt wurde isolierte chromosomale DNA von Listeria monocytogenes (siehe auch WO 90/08197) in Konzentrationen von 50 ng bis 5 fg pro Ansatz verwendet. NASBA ist beschrieben in EP-A- 0 329 822.

NASBA vorgeschaltet ist eine Reaktion zur Umwandlung der doppelsträngigen Analyt-DNA in Einzelstrang-DNA mit der $T_7$-RNA-Polymerase Promotorsequenz (PreNASBA). Diese wird im Normalfall mit Sequenase® durchgeführt. Im Prinzip ist aber jede DNA-Polymerase geeignet.

| **I PreNASBA-(Vorreaktion)** | **Endkonzentration in PreNASBA** |
|---|---|
| 10 X NASBA-Puffer | 1 X |
| Primer 3 | 0,2 µM |
| dATP (Pharmacia) | 1 mM |
| dCTP (Pharmacia) | 1 mM |
| dGTP (Pharmacia) | 1 mM |
| dTTP (Pharmacia) | 1 mM |
| ATP (Pharmacia) | 2 mM |
| CTP (Pharmacia) | 2 mM |
| GTP (Pharmacia) | 2 mM |
| UTP (Pharmacia) | 2 mM |
| dig11-UTP (Boehringer Mannheim) Best. Nr. 1209256) | 10-20 µM |
| + Target DNA | |
| PreNASBA-Mix | 20 µl |

Dieser Ansatz wurde 5 Minuten auf 95 °C erhitzt und auf Eis gekühlt.

Er wurde auf 10 mM Dithiothreitol (DTT) gebracht und mit 13 U $T_7$-DNA-Polymerase (Sequenase®, United States Biochemicals) versetzt. Es folgte eine 15minütige Inkubation bei 37 °C. Danach wurde erneut auf 95 °C erhitzt und wieder auf Eis gekühlt.

**IIA NASBA Amplifikation: Reaktionsführung 1**

a) Reaktionszusammensetzung

Das NASBA-Ansatzvolumen betrug 25 µl (23 µl NASBA-Mix + 2 µl PreNASBA-Mix, aus Beispiel 2, Teil I).

| Reagenz | Endkonzentration in NASBA |
|---|---|
| 10 X NASBA-Puffer | 1 X |
| DTT | 10 mM |
| dATP | 1 mM |
| dCTP | 1 mM |
| dGTP | 1 mM |
| dTTP | 1 mM |
| ATP | 2 mM |
| CTP | 2 mM |
| GTP | 2 mM |
| UTP | 2 mM |
| digUTP | 10-20 µM |
| RNase Inhibitor (Firma Boehringer Mannheim) | 0,48 U |
| $T_7$-RNA-Polymerase (Firma Boehringer Mannheim) | 80 U |
| AMV-RT (Firma Boehringer Mannheim) | 4 U |
| RNaseH (Firma Boehringer Mannheim) | 1 U |
| Primer 3 | 0,2 µM |
| Primer 4 | 0,2 µM |
| DMSO | 15 % |
| | 23 µl |
| + Produkt aus PreNASBA | 2 µl |
| | 25 µl |

10 x NASBA Puffer: 400 nM Tris/HCl, 500 mM KCl, 120 mM $MgCl_2$, pH 8,5

Primer 3: d(AATTCTAATACGACTCACTATAGGGAGACGCGCTTTACCTGCTT CGGCGATT), SEQ. ID. NO. 4 PIII-Region (Pos. 35-56). Der unterstrichene Teil ist die $T_7$-RNA-Polymerase Promotorsequenz.

Primer 4: d(GTAATCATCCGAAACCGCTCA), SEQ. ID. NO. 5 PIII-Region (Pos. 196c-216c)

Die NASBA-Amplifikation wurde für 1,5-2 Stunden bei 40 ° C durchgeführt.

b) Hybridisierung

Nach der Amplifikation wurde der NASBA-Ansatz mit 200 ng biotinylierter Sonde versetzt, für 5 min auf 95 ° C erhitzt und auf Eis gekühlt. Es folgte eine halbstündige Inkubation bei 37 ° C.

Sonde: d(CGTTTTACTTCTTGGACCG), SEQ. ID. NO. 6 Biotinyliert am 5' Ende mit Biotinamidit (Applied Biosystems) PIII-Region (Pos. 162c-180c)

c) Nachweis in Mikrotiterplatten

Zu 5 µl des Hybridisierungsansatzes wurden 195 µl Hybridisierungspuffer (50 mM Phosphatpuffer, 750 mM NACl, 75 mM Na-Citrat, 0,05 % RSA, pH 6,8) gegeben, die Mischung in eine Kavität einer Mikrotiterplatte pipettiert und eine weitere Stunde bei 37 ° C inkubiert. Nach dreimaligem Waschen mit 0,9 % NaCl-Lösung wurde mit 200 µl 0,2 U/ml Anti-Digoxigenin-POD (Boehringer Mannheim) in Konjugatpuffer (100 mM Tris-HCl, 0,9 % NaCl, 1 % RSA, pH 7,5) für 20 min bei 37 ° C inkubiert. Nach weiteren dreimal Waschen wurden 200 µl ABTS (Boehringer Mannheim) (1,9 mmol/l) zugegeben, und die Extinktion bei Hg 405 nm gemessen.

**IIB NASBA Amplifikation: Reaktionsführung 2**

a) Reaktionszusammensetzung

Die Zusammensetzung der NASBA-Reaktion entsprach IIA. Zusätzlich wurde die Fangsonde in einer Konzentration von 140 ng pro Ansatz direkt während der Amplifikation eingesetzt.

Für den direkten Einsatz der Fangsonde während der NASBA-Reaktion wurde ein 2'-O-Allylribooligonucleotid verwendet, das am 5'Ende mittels Biotin (Biotinamidit von Applied Biosystems Inc.) markiert und am 3' Ende mit einem hydrophilen Aminolinker blockiert ist. Ein 2'-O-Allyl-modifiziertes Oligoribonucleotid wurde als Sonde gewählt, da dieser "RNA-Strang" in RNA/DNA-Hybriden von RNAseH nicht abgebaut wird

(Lit.: Inoue, H et al. (1987) FEBS Lett. 215, 327-330). Das 3'-Ende des Oligonucleotids wurde blockiert, damit es nicht zu einer unspezifischen Verlängerung bei der Polymerase-Reaktion kommt.

Sequenz: 5'-Biotin-CGU UUU ACU UCU UGG ACC G-3'-Block (SEQ. ID. NO. 7)

C = 2'-O-Allylcytidin
G = 2'-O-Allylguanosin
A = 2'-O-Allyladenosin
U = 2'-O-Allyluridin

3'-Block:

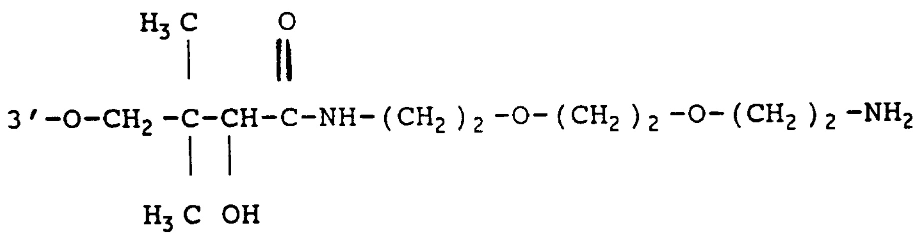

Die Synthese dieser Fangsonde erfolgte mit 2'-O-Allylnucleosidphosphoramiditen von Boehringer Mannheim Biochemica nach dem Protokoll von Iribarren et al. (Lit.: Iribarren, A.M. et al. (1990) PNAS 87, 7747-7751) auf einem Oligonucleotid-Synthesizer der Firma Pharmacia (Gene Assembler).

b) Hybridisierung

entfällt

c) MTP-Nachweis

Es wurde wie unter Ic beschrieben mit 5 µl NASBA-Ansatz vorgegangen.
Die Ergebnisse sind in Fig. 4 dargestellt.

Beispiel 3

Nachweis von HIV-1 durch NASBA

Im nachfolgenden Beispiel wurde HIV-1 mittels des Amplifikationssystems NASBA (EP-A-0 329 822) nachgewiesen. NASBA wurde in Gegenwart der mit Biotin markierten Fangsonde durchgeführt und das Amplifikat direkt durch den eingebauten Label (mit Ruthenium markiertes UTP) nachgewiesen.

Es wurden HIV-positive Seren von Patienten mit AIDS oder ARC (AIDS related complex) untersucht. Die RNA wurde analog der Guanidinium Hot Phenol-Methode nach Maniatis et al. [Molecular Cloning: A Laboratory Manual (Cold Spring Harbor Lab., Cold Spring Harbor, NY) 194-195, 1982] isoliert und die RNA-Lösungen in 10 µl-Aliquots bei -70 °C gelagert. Die RNA-Lösungen wurden vor der NASBA-Reaktion 1:10, 1:100, 1:1000, 1:10000 verdünnt und 2 µl in die NASBA-Reaktion eingesetzt.

NASBA-Reaktion und deren Zusammensetzung: s. Beispiel 2 IIb: Statt digUTP wurde 30 µM Ruthenium markiertes UTP eingesetzt (= $Ru(bpyr)_3$-AA-UTP) (Synthese siehe Beispiel 4).

Verwendete Primer und Sonde:

OT 188: AATTCTAATACGACTCACTATAGGGCCTGGCTTTAATTTTACTGGTA P1 (SEQ. ID. NO. 8)
OT 42: ACAGGAGCAGATGATACAGTATTAG P2 (SEQ. ID. NO. 9)
OT 15: 5'-Bio-UGGAAGAAAUCUGUUGACUCAGAUUGGUUGC-Block-3' Probe (SEQ. ID. NO. 10)

Der unterstrichene Teil der Sequenz OT 188 entspricht dem $T_7$-RNA-Polymerase Promotor.

Bei Oligonucleotid OT 15 handelt es sich wieder um ein 2'-O-Allylribooligonucleotid, das am 3' Ende blockiert (s. Beispiel 2 IIB) und am 5' Ende biotinyliert ist.

Lit.: Kievits, T. et al. (1991) J. of Virol. Methods 273-286.

**Nachweis der Amplifikate**

Nach der Reaktion wurden 5 µl NASBA-Ansatz mit 10 µg (2 x $10^6$ beads) Magnetic beads (Dynabeads$^R$ M-280, Streptavidin, Dynal) in 20 µl Phosphatpuffer (62 mM Na-Phosphat, 0,94 M NaCl, 94 mM Na-Citrat, pH 7) versetzt und 30 Minuten bei 37 °C inkubiert. Anschließend wurden die Beads mit Hilfe eines Magneten am Boden des Probencups abgeschieden und zweimal mit je 100 µl Phosphatpuffer gewaschen. Danach wurden die Beads in 100 µl des soeben genannten Phosphatpuffers suspendiert und 250 µl ECL-Assay-Puffer (0,2 M $KH_2PO_4$, 0,05 M NaCl, 0,1 M Tripropylamin, pH 7,5; Lit.: J.K. Leland et al. J. Electrochem. Soc. 1990, 137, 3127-33) zugefügt. Gemessen wurde auf einem ECL-Analyser (Origen 1.0; Igen Inc.) bei einer Photomultiplierspannung von 940 V. Die Messzeit betrug 2 Sekunden bei einer Rampe von 2 V pro Sekunde. Der gesamte Meßzyklus pro Probe betrug 2 Minuten.

Das Ergebnis ist in Fig. 5 dargestellt.

Beispiel 4

Synthese von $Ru(bpyr)_3$-AA-UTP

Origen$^R$ label (Igen Inc) = [4-(N-succinimidyloxycarbonylpropyl)-4'-methyl-2,2'-bipyridine-bis(2,2'-bipyridine)]-ruthenium (II)-dihexafluorophosphate

AA-UTP = 5-(3-Aminoallyl)-Uridinetriphosphat wurde analog Langer et al. [Proc. Natl. Acad. SCI. USA, 78, 6633-37, 1981] hergestellt.

DMSO = Dimethylsulfoxid

5,5 mg (0,01 mMol) AA-UTP (Lithium-Salz) wurden in 2 ml 0,1 M Natriumborat-Puffer (pH 8,5) gelöst. Dazu gab man eine Lösung aus 15,8 mg (0,015 mMol) des Origen® labels in 1,5 ml DMSO und ließ über Nacht bei Raumtemperatur rühren. Anschließend wurde das Produkt Ru $(bpyr)_3$-AA-UTP durch Ionenaustausch-chromatographie gereinigt (Sephadex DEAE, C1-Form; Gradient: 0-0, 4M LiCl). Ausbeute: 7 mg (46 % d.Th.)

Beispiel 5:

Synthese von $Ru(bpyr)_3$-AA-dUTP und $Ru(bpyr)_3$-DADOO-dUTP

5-(3-Aminoallyl)-dUTP (= AAdUTP) wurde analog Langer et al. Proc. Natl. Acad. Sci. USA, 78, 6633-37, 1981 hergestellt, DADOO-dUTP durch Umsetzung von 5-Mercury-dUTP mit N-Allyl-N'-(8-amino-3, 5-dioxa-octyl)harnstoff.

Synthese von $Ru(bpyr)_3$-AA-dUTP (FIG 7):

3,2 mg AAdUTP wurden in 0,1 M Natriumboratpuffer gelöst und über Nacht bei RT mit einer Lösung aus 5,6 mg des Origen® labels in 560 µl DMSO gerührt.

Die Umsetzung wurde mittels Dünnschichtchromatographie und Papierelektrophorese verfolgt und das Produkt anschließend durch Ionenaustauschchromatographie gereinigt.

Ausbeute: 3,8 mg (48 % d.Th.)

$[M + H]^+$: 1180.1

Synthese von $Ru(bpyr)_3$ -DADOO-dUTP (FIG 8):

a) Synthese von N-Allyl-N'-(8-amino-3,5-dioxaoctyl)harnstoff

15 g (0.1 mol) Diaminodioxaoctan (Merck) werden in 500 ml Diisopropylether auf -40 °C gekühlt. Dazu tropft man langsam 5,5 g (0.06 mol) Allylisocyanat (Fluka). Nach 30 min Rühren bei -40 °C läßt man den Ansatz auf RT kommen und entfernt Diisopropylether durch Destillation. Der Rückstand wird 1 h mit 600 ml Essigester ausgerührt, der Niederschlag abfiltriert und dreimal mit Essigester nachgewaschen. Die vereinigten Essigesterfraktionen werden konzentriert und das Produkt mittels Flash-Chromatographie mit einem Lösungsmittelgradient aus Essigester/methanol = 9:1 bis 8:2 gereinigt.

Ausbeute: 4,5 g (29 % d.Th.) farblose Flüssigkeit.

b) Umsetzung mit 5-Mercury-dUTP

5-Mercury-dUTP (= HgdUTP) wurde nach Bergstrom et al., J Carbohydr., Nucleosides, Nucleotides 4, 257 (1977) hergestellt.

370 mg (0.54 mmol) HgdUTP und 113 mg $K_2PdCl_4$ werden in 20 ml Natriumacetatpuffer (ph = 5) gelöst. Dazu tropft man 0.5 g (2.16 mmol) N-Allyl-N'-(8-amino-3,5-dioxaoctyl)harnstoff und läßt über Nacht bei RT rühren. Der Niederschlag wird abfiltriert, das Filtrat eingeengt und durch Ionenaustauschchromatographie (DEAE-Sephadex-CL-Form) mit einem Puffergradienten von 0 bis 0,3 M LiCl gereinigt.

Anschließend wird das Produkt zweimal aus Aceto/etanol = 2:1 gefällt, der Niederschlag abfiltriert, gewaschen und getrocknet. Man erhält 60 mg (15,7 % d.Th.) DADOO-dUTP.
c) Die Synthese erfolgte analog zur Darstellung von $Ru(bpyr)_3$-AA-dUTP durch Umsetzung von DADOO-dUTP mit dem Origen® label.

Beispiel 6

PCR-Amplifikation und Markierung von HBV-DNA mit $Ru(bpyr)_3$-AA-dUTP und $Ru(bpyr)_3$-DADOO-dUTP

Amplifiziert wurde jeweils 1 ng Hpbadw21, das PCR-Ansatzvolumen betrug 100 µl (80 µl PCR-Mix, 20 µl Probe).

Der PCR-Mix setzte sich wie folgt zusammen:

| Reagenz oder Probe | Konzentration der Vorratslösung | Endkonzentration in der PCR |
|---|---|---|
| Hpbadw211 | | ng/100 µl |
| Primer 1 | 5 µM | 200 nM |
| Primer 2 | 5 µM | 200 nM |
| dATP (Li-Salz) | 4 mM | 200 µM |
| dCTP (Li-Salz) | 4 mM | 200 µM |
| dGTP (Li-Salz) | 4 mM | 200 µM |
| dTTP (Li-Salz) | 4 mM | 133 µM - 200 µM |
| $Ru(bpy)_3$-AA-dUTP | 700 µM | 66 µM - 0.01 µM |
| oder | | |
| $Ru(bpy)_3$- DADOO-dUTP | 300 µM | 66 µM - 0.01 µM |
| PCR-Puffer | 10 x | 1 x |
| Taq-Polymerase | 5 U/µl | 2,5 U |

10 x PCR-Puffer: 100 mM Tris/HCl, 500 mM KCl, 15 mM $MgCl_2$, 100 mg/ml Gelatine, pH 9,0
Primer 1: Oligodesoxynucleotid, 18 mer, d(GGAGTGTGGATTCGCACT) SEQ. ID. NO. 1 (Pos. 2267-2284; EMBL, subtyp adw)
Primer 2: Oligodesoxynucleotid, 18 mer, d(TGAGATCTTCTGCGACGC) SEQ. ID. NO. 2 (Pos. 2436c-2419c; EMBL; subtyp adw)
Hpbadw 21: adw 21 cloniert in pUC BM 20 (Boehringer Mannheim) und linearisiert

Die PCR-Ansätze wurden in einem Perkin Elmer Thermal Cycler mit 30 Cyclen von 60 sec bei 94 °C, 60 sec bei 50 °C und 120 sec bei 70 °C amplifiziert.

Die Konzentration von $Ru(bpyr)_3$-AA-dUTP und $Ru(bpyr)_3$-DADOO-dUTP wurde von 66 µM, 30 µM, 10 µM, 1 µM, 0.1 µM bis 0.01 µM variiert, die Konzentration an dTTP entsprechend heraufgesetzt, so daß die Gesamtkonzentration an $Ru(bpyr)_3$dUTP/dTTP im Amplifikationsansatz 200 µM betrug.

Als Vergleich wurde der gleiche PCR-Ansatz unter Einbau verschiedener Konzentrationen von Digoxigenin-11-dUTP (Boehringer Mannheim, Best. Nr. 1093 088) sowie ein PCR-Ansatz ohne Einbau von modifizierten Nukleotiden durchgeführt.

| No. | dTTP uM | $Ru(bpyr)_3$ dUTP (short) uM | No. | dTTP uM | $Ru(bpyr)_3$ dUTP (long) uM | No. |
|---|---|---|---|---|---|---|
| 1 | 133 | 66 | 1/1 | 133 | 66 | 1/2 |
| 2 | 170 | 30 | 2/1 | 170 | 30 | 2/2 |
| 3 | 190 | 10 | 3/1 | 190 | 10 | 3/2 |
| 4 | 199 | 1 | 4/1 | 199 | 1 | 4/2 |
| 5 | 200 | 0.1 | 5/1 | 200 | 0.1 | 5/2 |
| 6 | 200 | 0.01 | 6/1 | 200 | 0.01 | 6/2 |

| No. | dTTP uM | DigdUTP uM | No. | dTTP uM |
|---|---|---|---|---|
| 1 | 133 | 66 | 1/3 | 133 |
| 2 | 170 | 30 | 2/3 | 170 |
| 3 | 190 | 10 | 3/3 | 190 |
| 4 | 199 | 1 | 4/3 | 200 |
| 5 | 200 | 0.1 | | |
| 6 | 200 | 0.01 | | |

| Bahn | Ansatz No. | Bahn | Ansatz No. |
|---|---|---|---|
| I | 1 | XIII | 4 |
| II | 1/1 | XIV | 4/1 |
| III | 1/2 | XV | 4/2 |
| IV | 1/3 | XVI | 4/3 |
| V | 2 | XVII | 5 |
| VI | 2/1 | XVIII | 5/1 |
| VII | 2/2 | IXX | 5/2 |
| VIII | 2/3 | XX | 6 |
| IX | 3 | XXI | 6/1 |
| X | 3/1 | XXII | 6/2 |
| XI | 3/2 | | |
| XII | 3/3 | | |

Jeweils 10 µl der verschiedenen PCR-Ansätze wurden auf ein 1%iges Agarosegel aufgetragen und die Amplifikate durch Anfärben mit Ethidiumbromid sichtbar gemacht (FIG 9). Das Amplifikationsprodukt mit eingebautem $Ru(bpyr)_3$-DADOO-dUTP (= langer Spacer) hat aufgrund des höheren Molekulargewichtes gegenüber dem entsprechend mit $Ru(bpyr)_3$-AA-dUTP (= kurzer Spacer) markierten Produktes eine geringere Wanderungsgeschwindigkeit im Gel. Dies ist jedoch nur bei den höheren Konzentrationen an modifiziertem Nukleotid zu beobachten, da entsprechend mehr Label während der Amplifikationsreaktion eingebaut werden kann und sich das Molekulargewicht gegenüber dem unmodifizierten Amplifikat erheblich vergrößert.

Beispiel 7

Filterhybridisierung mit biotinylierter Oligonukleotidsonde

Biotinylierte Fangsonde:
d(AGA CCA CCA AAT GCC CCT AT) SEQ. ID. NO. 11 biotinyliert mit Biotinamidit (Applied Biosystems), entspricht der Pos. 2297-2316 der $HBV_{adw}$-Sequenz; EMBL-Sequenzdatenbank, Lit.: Ono et al., Nucl. Acids Res. 11 (1983) 1747-1757.

10 µl der PCR-Ansätze 1 - 6, 1/1 - 6/1 und 1/3 - 2/3 wurden auf ein 1%iges Agarosegel aufgetragen, anschließend auf eine Nylonmembran geblottet und mit je 100 ng der oben beschriebenen biotinylierten Oligonukleotidsonde bei 45°C analog Ch. Kessler et al. "Non-radioactive labelling and detection of nucleic acids", Biol. Chem. Hoppe-Seyler, 1980, 371, 917-927, über Nacht hybridisiert. Biotin wurde mit einem Konjugat aus Streptavidin und alkalischer Phosphatase (Boehringer Mannheim, Best. Nr. 1093 266), NBT/X-Phosphat (Boehringer Mannheim, Best. Nr. 1383 213 und 760 986) nachgewiesen. Wie FIG 10 zeigt, konnte bei allen PCR-Ansätzen das gewünschte Amplifikat duch Hybridisierung mit einer für das Amplifikationsprodukt spezifischen Oligonukleotidsonde nachgewiesen werden.

Beispiel 8

Hybridisierung und Detektion

4.1 "Zwei-Schritt-Assay"
10 µl der Ansätze 1,4 und 6 sowie 1/1, 4/1 und 6/1 wurden 1 : 10 mit 0.05 M Na0H verdünnt. 100 µl

dieses Ansatzes wurden nach 5 min mit 400 µl Hybridisierungspuffer (62,5 mM Na-Phosphat, 0,94 M NaCl, 94 mM Na-Citrat, pH = 6,5), der bereits die biotinylierte Oligonukleotidsonde in einer Konzentration von 94 ng/ml enthält, neutralisiert. Parallel dazu erfolgte eine thermische Denaturierung der PCR-Ansätze 1, 4 und 6 sowie 1/1, 4/1 und 6/1 durch 2 min Erhitzen auf 100 °C und anschließend Abkühlen der Probe auf Eis. Nach 1 h Hybridisierung bei 37 °C gab man 50 µl einer Suspension von Streptavidin beschichteten Magnetpartikel (Dynabeads® M-280, Streptavidin, Dynal) in Hybridisierungspuffer (Konzentration 600 µg/ml) zu und inkubierte nochmals für 1 h bei 37 °C. Mit Hilfe eines Magneten wurden die Magnetbeads am Boden des Probencups abgeschieden und die überstehende Lösung abgegossen und die Beads nochmal 2 mit je 500 µl Phosphatpuffer (50 mM Na-Phosphat, pH = 7,4) gewaschen. Zuletzt wurden die Beads in 200 µl Phosphatpuffer suspendiert, 500 µl ECL-Assay-Puffer (siehe Blackburn et al., 1991, Clin. Chem. 37, 1534-1539) zugefügt und auf einem ECL-Analyzer (Origen 1.0®, IGEN, Rockville, USA) gemessen. Gemessen wurde bei einer Photomultiplierspannung von 940 V. Die Meßzeit betrug 2 sek bei einer Rampe von 2 V pro Sekunde. Der gesamte Meßzyclus pro Probe betrug 2 Minuten.

FIG 11 zeigt die gemessenen ECL-Peakintensitäten in Abhängigkeit unterschiedlicher Konzentrationen von $Ru(bpyr)_3$-AA-dUTP und $Ru(bpyr)_3$-DADOO-dUTP im Amplifikationsansatz.

4.2 "Ein-Schritt-Assay"

Bei dieser zweiten und einfacheren Testführung wurde die Denaturierung des PCR-Ansatzes und Hybridisierung mit der biotinylierten Fangsonde analog dem oben beschriebenen Zwei-Schritt-Assay durchgeführt. Nach 2 h Hybridisierung bei 37 °C wurden wiederum 50 µl Magnetbeads zupipettiert und dann jedoch ohne "bound-free"-Trennung direkt im ECL-Analyzer gemessen. Als Leerwert wurde jeweils die entsprechende Konzentration an $Ru(bpyr)_3$-AA-dUTP in Hybridisierungspuffer gemessen.

Die Photomultiplierspannung betrug 800 V, ansonsten wurden analoge Meßbedingungen wie oben beschrieben verwendet.

FIG 12 zeigt wiederum die gemessenen ECL-Peakintensitäten in Abhängigkeit unterschiedlicher Konzentrationen von $Ru(bpyr)_3$-AA-dUTP und $Ru(bpyr)_3$-DADOO-dUTP im Amplifikationsansatz.

## Beispiel 9

### Nachweis von HBV-Plasmid-DNA

Als Target wurde wiederum Hpbadw 21 mit folgenden Konzentrationen verwendet: 1 ng; 10 pg; 100 fg; 1 fg; 200 ag. Die Amplifikation erfolgte analog den in Beispiel 2 beschriebenen Bedingungen, einmal unter Verwendung von 10 µM $Ru(bpyr)_3$-AA-dUTP/190 µl dTTP und einmal unter Verwendung von 30 µM $Ru(bpyr)_3$-AAdUTP/170 µl dTTP Endkonzentration in der PCR. Das Amplifikationsprodukt wurde, wie unter 4.2 beschrieben, nach Hybridisierung mit der biotinylierten Fangsonde (Seq. Id. Nr. 3) und Bindung an SA-Magnetbeads in der "Einschritt-Testführung" ohne "bound- free"-Trennung im Origen®-1.0-Analyzer gemessen. Die Photomultiplierspannung betrug 900 V, die übrigen Bedingungen waren wie in Beispiel 4.1 beschrieben.

FIG 13 zeigt die gemessenen ECL-Peakintensitäten in Abhängigkeit der unterschiedlichen Targetkonzentrationen.

## Beispiel 10

### HBV-Nachweis mit direktem Einsatz der Fangsonde während der PCR in mit thermostabilem SA-beschichteten Mikrotiterplatten

Der PCR-Ansatz wird wie in Beispiel 1 beschrieben durchgeführt. Nur das Ansatzvolumen wurde auf 50 µl reduziert und mit 30 µl Sigma Mineralöl (Sigma Mineral oil, light white oil M-3516) überschichtet. Die verwendeten Primer, biotinylierte Fangsonde II und das Template sowie dessen Konzentrationen sind identisch. Die PCR wurde in Techne-MT-Platten (Best.nr.: 140800), die zuvor mit thermostabilem Streptavidin (SA) beschichtet wurden, durchgeführt. Amplifiziert wurde in einem Techne-Cycler Typ MW-2. Die Cyclebedingungen waren die gleichen wie in Beispiel 1 beschrieben. Der Ansatz wurde nach der PCR 30 min bei 37 °C stehen gelassen. Dann wurde dreimal gewaschen (s. Beispiel 1) und die Platte mit 100 µl Konjugatpuffer (s. Beispiel 1) pro well inkubiert. Nach erneutem dreimaligem Waschen erfolgte die Umsetzung mit 100 µl. ABTS Lösung (s. Beispiel 1). Die gemessene Extinktion ist in FIG. 14 als jeweils der rechte Balken (SA-MTP PCR) für verschiedene Konzentrationen gezeigt.

Beispiel 11

HBV-Nachweis mit direktem Einsatz der Fangsonde während der PCR in mit thermostabilem SA-beschichteten Tubes (Cups)

Verwendet wurden 0,5 ml Perkin-Elmer Cups (Best.nr.: N801.0537) für den PCR-Cycler 9600, die gemäß EP-B-0 331 127 innen mit thermostabilem Streptavidin beschichtet worden waren. Der Ansatz wurde wie in Beispiel 10 durchgeführt. Das Ansatzvolumen betrug jedoch 100 µl und die PCR wurde wegen des beheizbaren Deckels des Cyclers 9600 nicht mit Öl überschichtet. Konjugatpuffer und ABTS wurden je 200 µl pro Cup zugesetzt. Nach 15 minütiger Inkubation mit ABTS wurde der Ansatz in eine Nunc-Platte (Best.nr.: 838713) überführt und im Reader vermessen. Die gemessenen Extinktionen sind in FIG. 14 (mittlerer Balken) gezeigt.

Beispiel 12

Beschichtung der Techne-Platten und der EP-Cups mit thermostabilem Streptavidin

Die Beschichtungen wurden nach EP-B-0 331 127 vorgenommen.

## Patentansprüche

1. Verfahren zur Immobilisierung von Nukleinsäuren durch Hybridisierung der Nukleinsäure mit einer Fangsonde, dadurch gekennzeichnet, daß die Fangsonde gegen enzymatische Verlängerung oder/und enzymatischen Abbau des gebildeten Hybrids geschützt ist.

2. Verfahren zum Nachweis einer Nukleinsäure durch
   - Immobilisierung der Nukleinsäure durch Hybridisierung mit einer Fangsonde und

- Nachweis des immobilisierten Hybrids

dadurch gekennzeichnet, daß die Fangsonde gegen enzymatische Verlängerung oder/und enzymatischen Abbau geschützt ist.

3. Verfahren zum Nachweis von Nukleinsäuren durch
   - Bildung einer oder mehrerer detektierbar markierter Kopien der Nukleinsäuren oder Teilen davon;
   - Immobilisierung der Kopien durch Hybridisierung mit einer Fangsonde und
   - Nachweis des immobilisierten Hybrids,

   dadurch gekennzeichnet, daß die Fangsonde gegen enzymatische Verlängerung oder/und enzymatischen Abbau geschützt ist.

4. Verfahren gemäß Anspruch 3, dadurch gekennzeichnet, daß die Fangsonde schon während der Bildung der Kopien anwesend ist.

5. Verfahren gemäß Anspruch 4, dadurch gekennzeichnet, daß auch eine Nachweissonde schon während der Bildung der Kopien anwesend ist.

6. Verfahren gemäß Anspruch 2 oder 3, dadurch gekennzeichnet, daß die Kopien bei ihrer Bildung detektierbar markiert werden.

7. Verfahren nach Anspruch 1, dadurch gekennzeichnet, daß das 3'-Ende der Fangsonde gegen Verlängerung geschützt ist.

8. Verfahren nach Anspruch 6, dadurch gekennzeichnet, daß die nachweisbare Markierung über ein nachweisbar markiertes Mononukleosidtriphosphat eingebaut wird.

9. Verfahren nach Anspruch 7, dadurch gekennzeichnet, daß die nachweisbare Markierung ein durch Anlegen eines elektrischen Feldes anregbarer Rest ist.

10. Reagenzkit zur Amplifikation und Immobilisierung von Nukleinsäuren enthaltend
    - einen Behälter A mit einem Enzym zur Verlängerung von Nukleinsäuren
    - einen Behälter B mit einer Fangsonde,

    dadurch gekennzeichnet, daß die Fangsonde gegen enzymatische Verlängerung oder/und enzymatischen Abbau geschützt ist.

11. Reagenzkit zum Nachweis von Nukleinsäuren enthaltend ein Reagenzkit gemäß Anspruch 10 und ferner eine detektierbare Nachweissonde oder detektierbar markierte Mononukleosidtriphosphate.

12. Modifizierte Nukleinsäure, die gegen enzymatische Verlängerung und enzymatischen Abbau geschützt ist, dadurch gekennzeichnet, daß sie an den zur Wasserstoffbrückenbindung zu komplementären Nukleinsäuren fähigen Positionen der Nukleobasen nicht gegenüber den natürlichen Nukleobasen modifiziert ist.

13. Mononukleosidtriphosphat der Formel I

    P-Z-B-E (I),

    in der

    | | |
    |---|---|
    | P | ein Triphosphatrest oder triphosphatanaloger Rest, |
    | Z | ein Zucker oder zuckeranaloger Rest, |
    | B | eine Nukleobase oder nukleobasenanaloger Rest und |
    | E | ein eine Elektrochemilumineszenzgruppe enthaltender Rest |

    ist.

14. Triphosphat nach Anspruch 13, gekennzeichnet durch die Strukturformel Ia

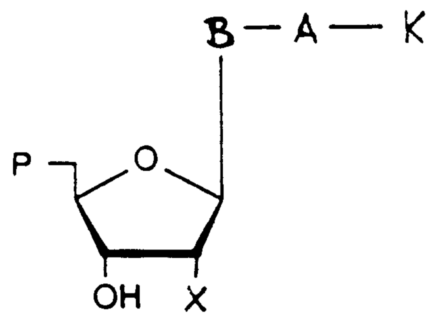

wobei

- x Wasserstoff oder die Gruppe $OR^1$,
- $R^1$ ein $C_1$-$C_6$ -Alkylrest, $C_1$-$C_6$-Alkylenrest oder Wasserstoff
- B ein Purin-, Deazapurin- oder Pyrimidinrest ist
- A Eine Abstandsgruppe und
- K ein elektrochemilumineszenter Koordinationskomplex ist

sowie deren Alkali- oder Erdalkalisalze.

**15.** Triphosphat nach einem der Ansprüche 13 und 14, dadurch gekennzeichnet, daß die Elektrochemilumineszenzgruppe ein Koordinationskomplex eines Metallions und einem oder mehreren organischen Liganden eines Atomgewichts von mehr als 500 g/mol ist.

**16.** Triphosphat nach Anspruch 5, dadurch gekennzeichnet, daß das Molekulargewicht des Restes E zwischen 550 und 2000 g/mol liegt.

Fig. 1

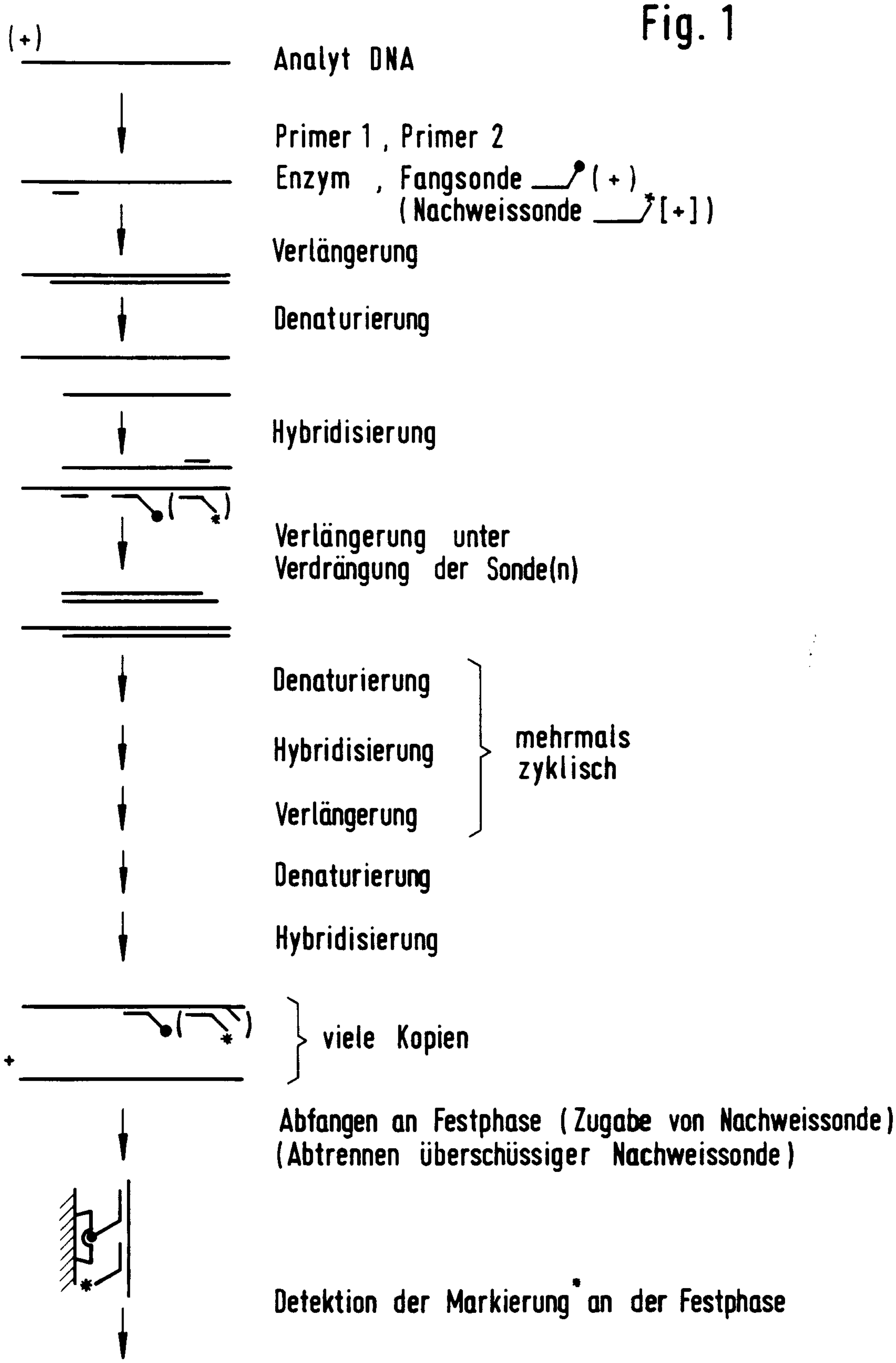
(+)
Analyt DNA
Primer 1, Primer 2
Enzym, Fangsonde (+)
(Nachweissonde [+])
Verlängerung
Denaturierung
Hybridisierung
Verlängerung unter
Verdrängung der Sonde(n)
Denaturierung
Hybridisierung
Verlängerung
mehrmals
zyklisch
Denaturierung
Hybridisierung
viele Kopien
+
Abfangen an Festphase (Zugabe von Nachweissonde)
(Abtrennen überschüssiger Nachweissonde)
Detektion der Markierung* an der Festphase

Fig. 2

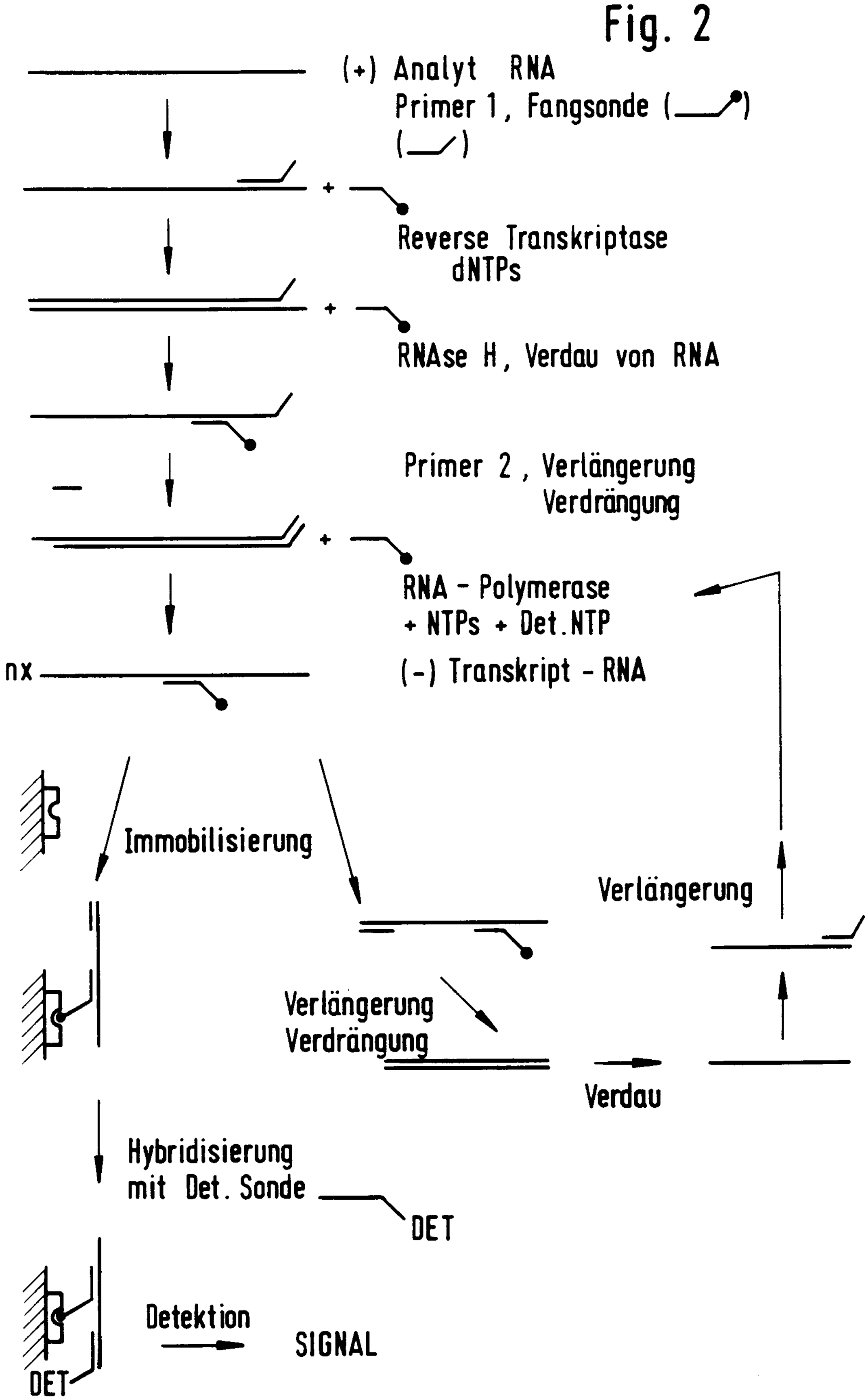
(+) Analyt RNA
Primer 1, Fangsonde
Reverse Transkriptase
dNTPs
RNAse H, Verdau von RNA
Primer 2, Verlängerung
Verdrängung
RNA - Polymerase
+ NTPs + Det.NTP
nx
(-) Transkript - RNA
Immobilisierung
Verlängerung
Verlängerung
Verdrängung
Verdau
Hybridisierung
mit Det. Sonde
DET
DET
Detektion
SIGNAL

Fig. 3

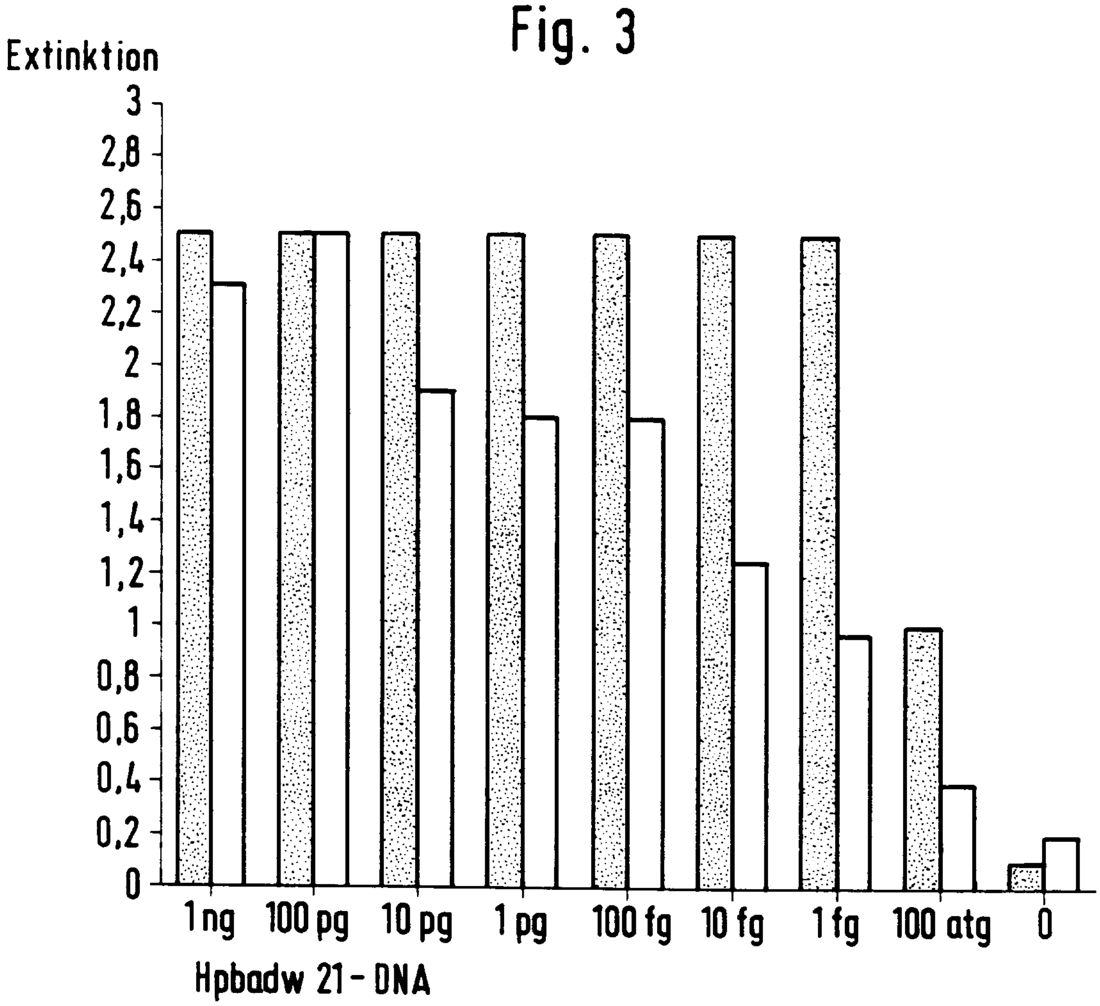
Extinktion
3
2,8
2,6
2,4
2,2
2
1,8
1,6
1,4
1,2
1
0,8
0,6
0,4
0,2
0
1 ng
100 pg
10 pg
1 pg
100 fg
10 fg
1 fg
100 atg
0
Hpbadw 21 - DNA

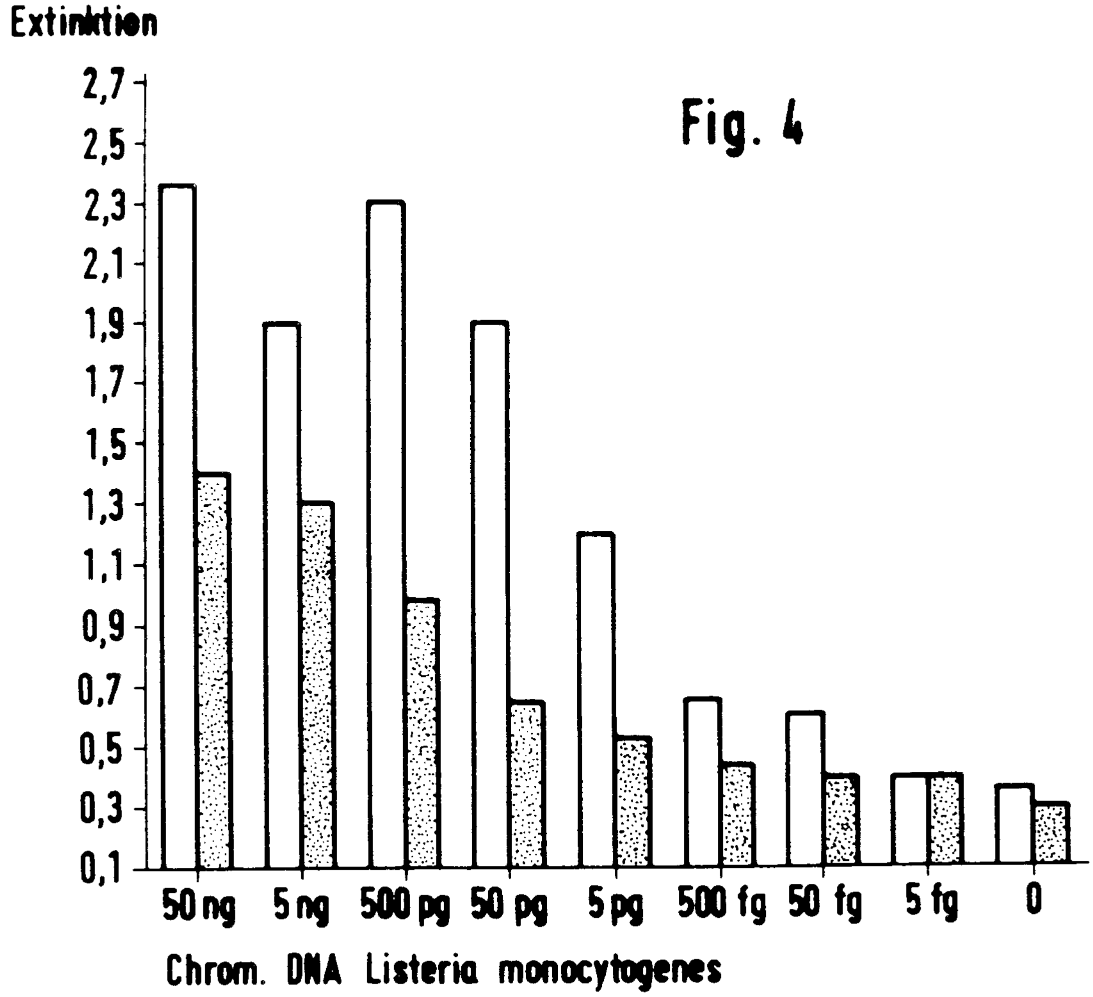
Extinktion
2,7
2,5
2,3
2,1
1,9
1,7
1,5
1,3
1,1
0,9
0,7
0,5
0,3
0,1
50 ng
5 ng
500 pg
50 pg
5 pg
500 fg
50 fg
5 fg
0
Chrom. DNA Listeria monocytogenes

Fig. 4

Fig. 5

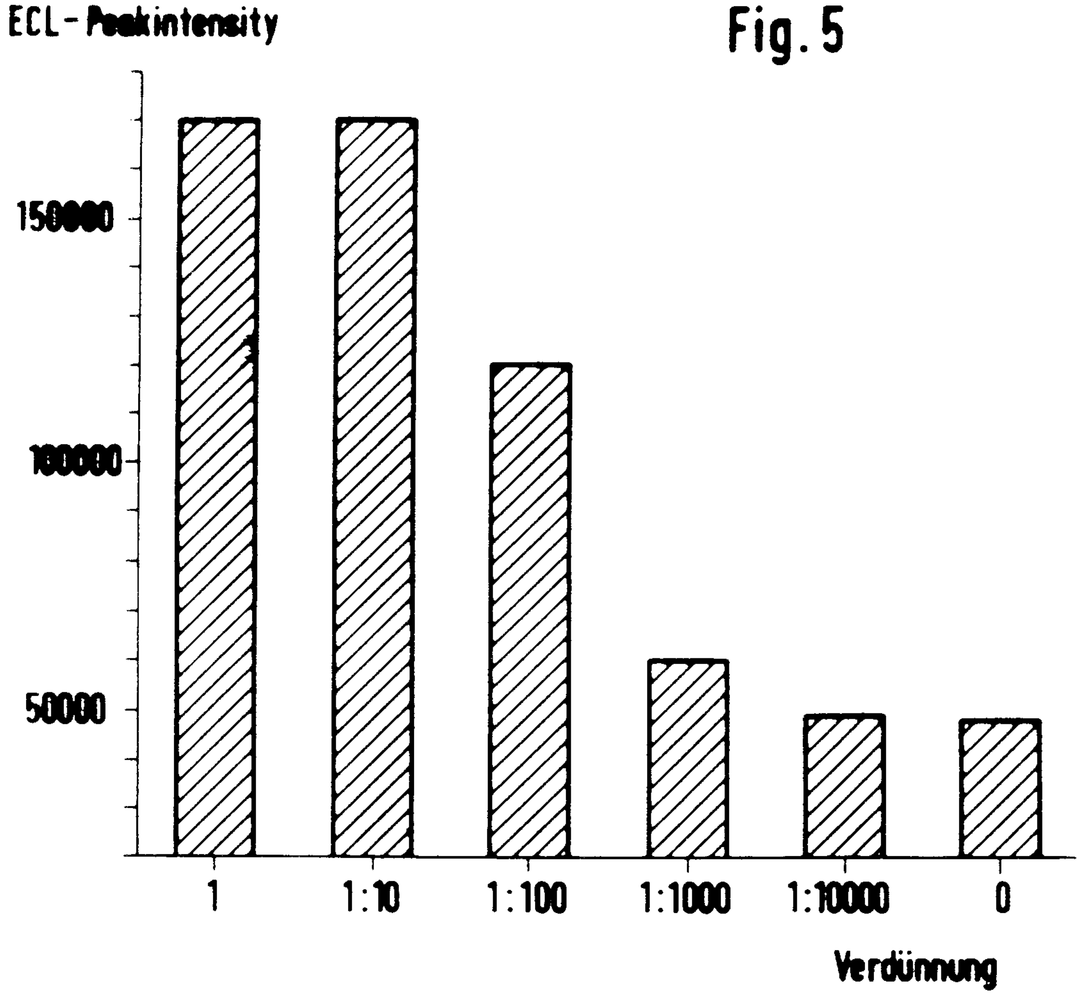
ECL-Peakintensity
150000
100000
50000
1
1:10
1:100
1:1000
1:10000
0
Verdünnung

Fig. 6

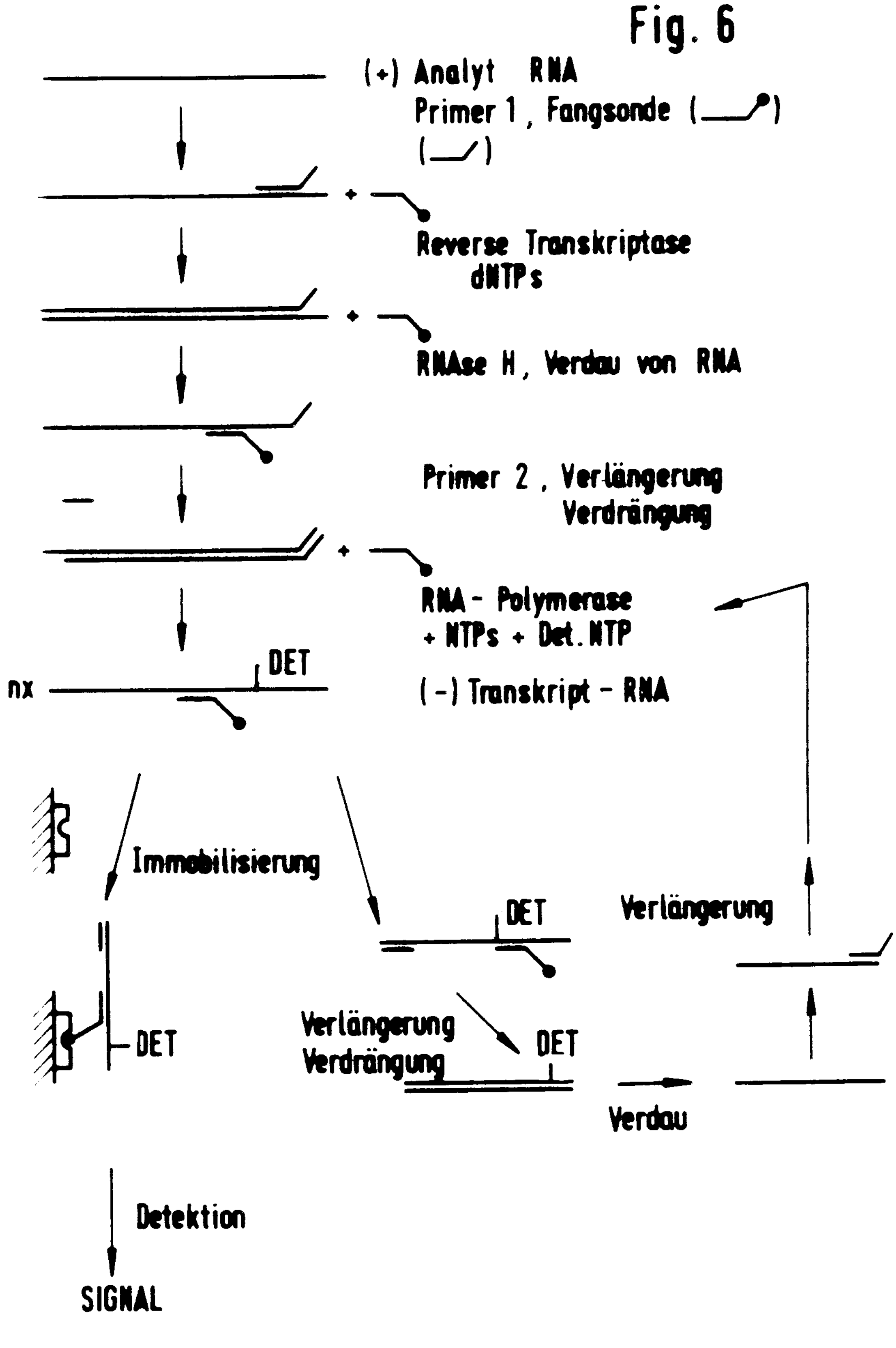
(+) Analyt RNA
Primer 1, Fangsonde
Reverse Transkriptase
dNTPs
RNAse H, Verdau von RNA
Primer 2, Verlängerung
Verdrängung
RNA - Polymerase
+ NTPs + Det. NTP
nx
DET
(-) Transkript - RNA
Immobilisierung
DET
Verlängerung
DET
Verlängerung
Verdrängung
DET
Verdau
Detektion
SIGNAL

FIG 7

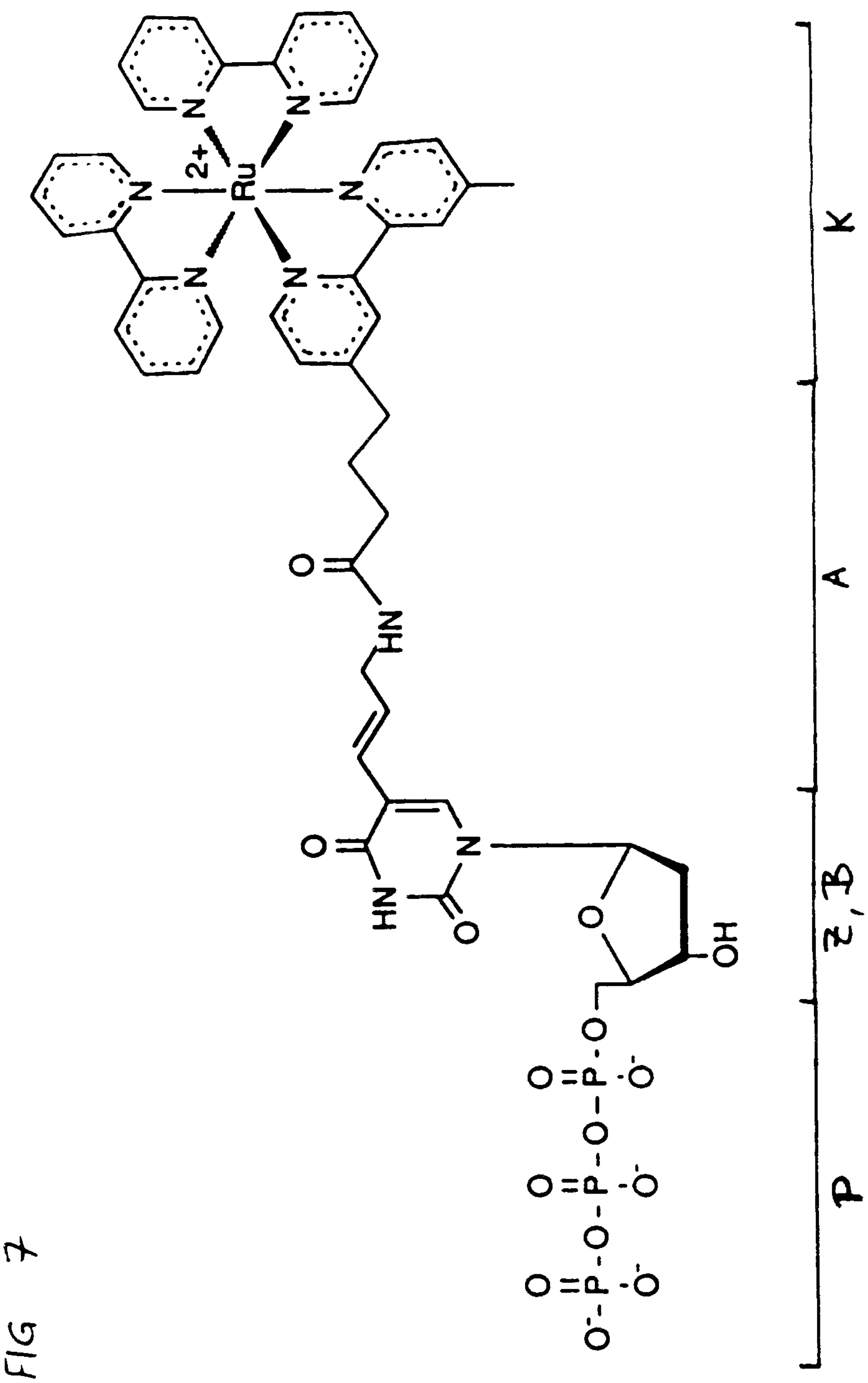

FIG 8

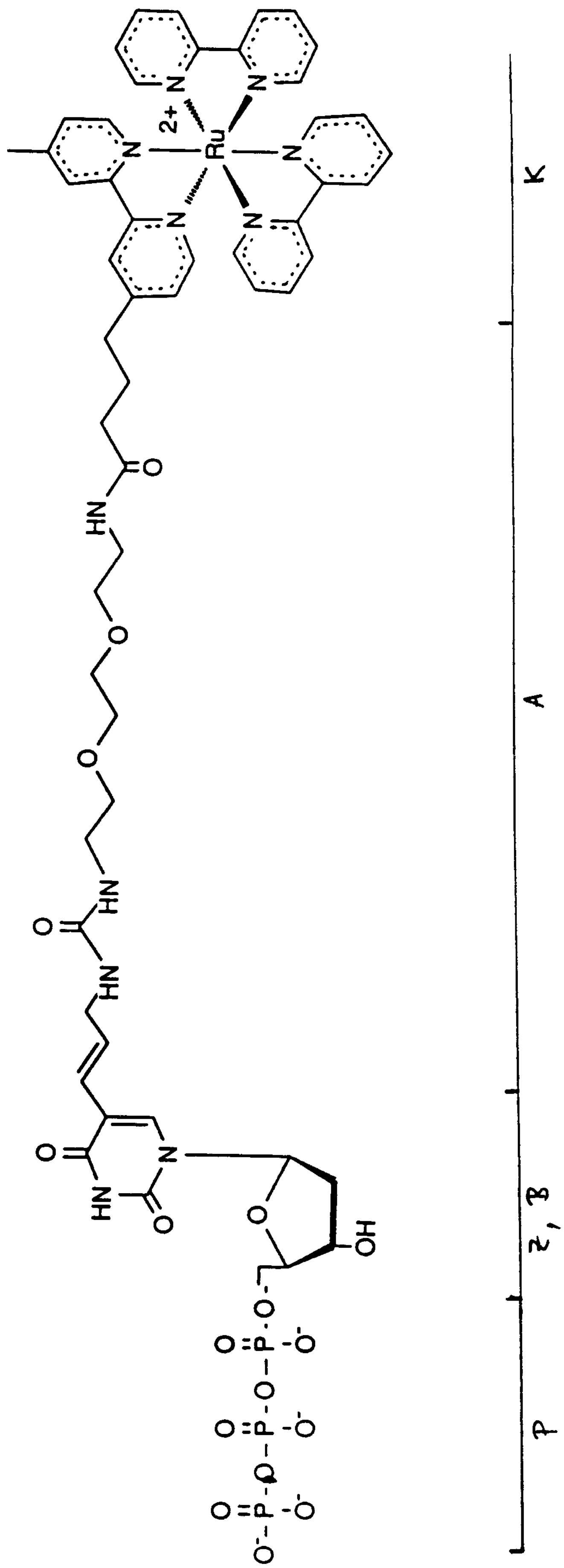

K

A

Z, B

P

FIG 9

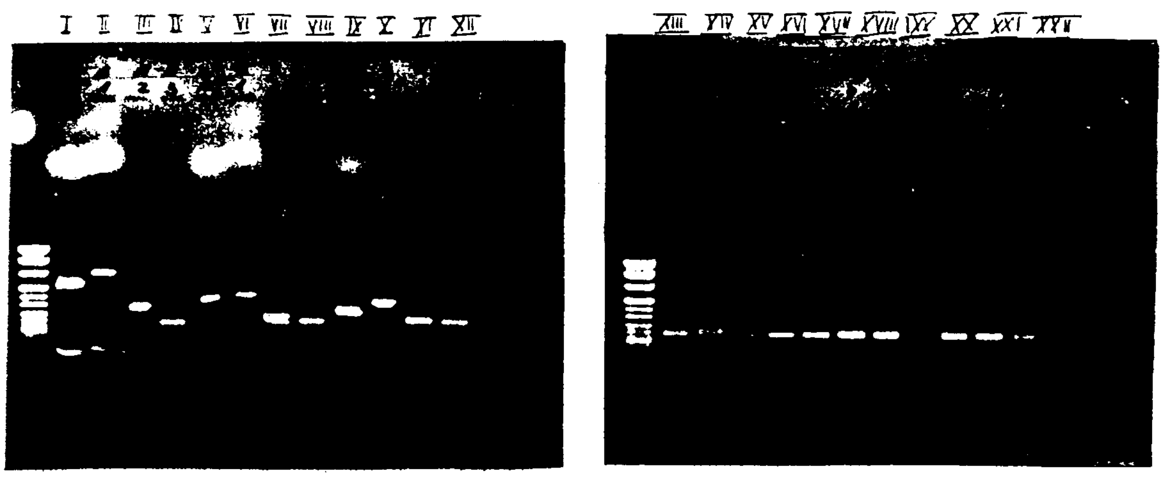

FIG 10

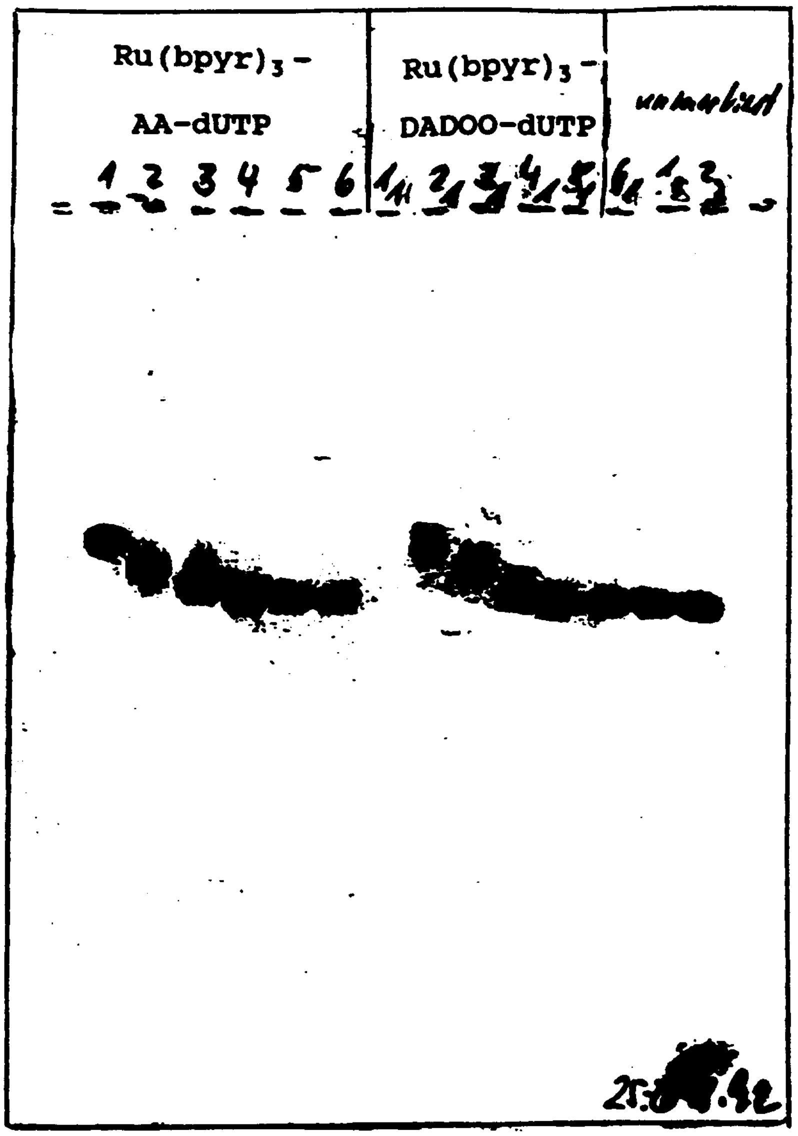

FIG 11

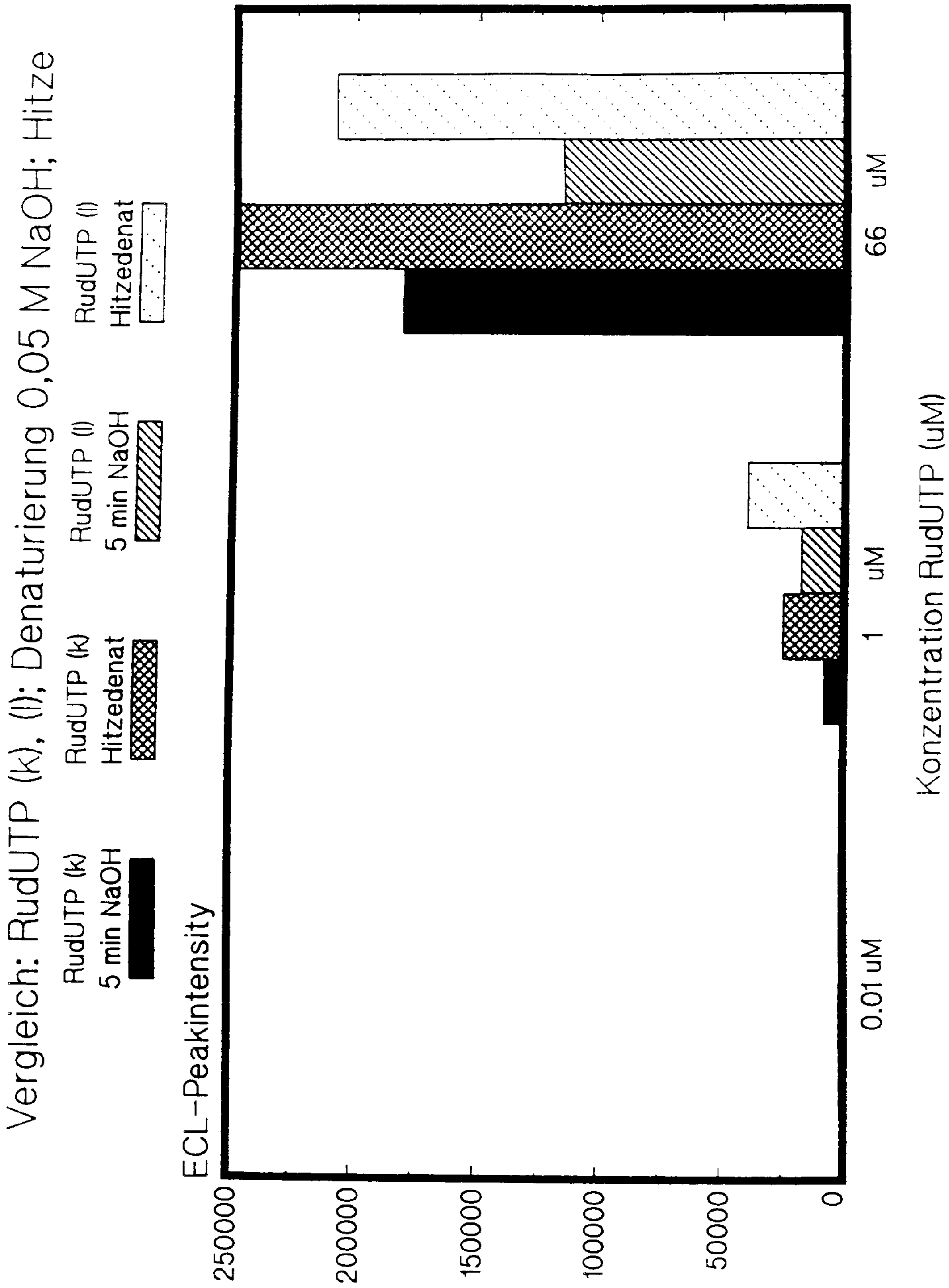
Vergleich: RudUTP (k), (l); Denaturierung 0,05 M NaOH; Hitze
RudUTP (k) 5 min NaOH
RudUTP (k) Hitzedenat
RudUTP (l) 5 min NaOH
RudUTP (l) Hitzedenat
ECL-Peakintensity
250000
200000
150000
100000
50000
0
0.01 uM
1 uM
66 uM
Konzentration RudUTP (uM)

FIG 12

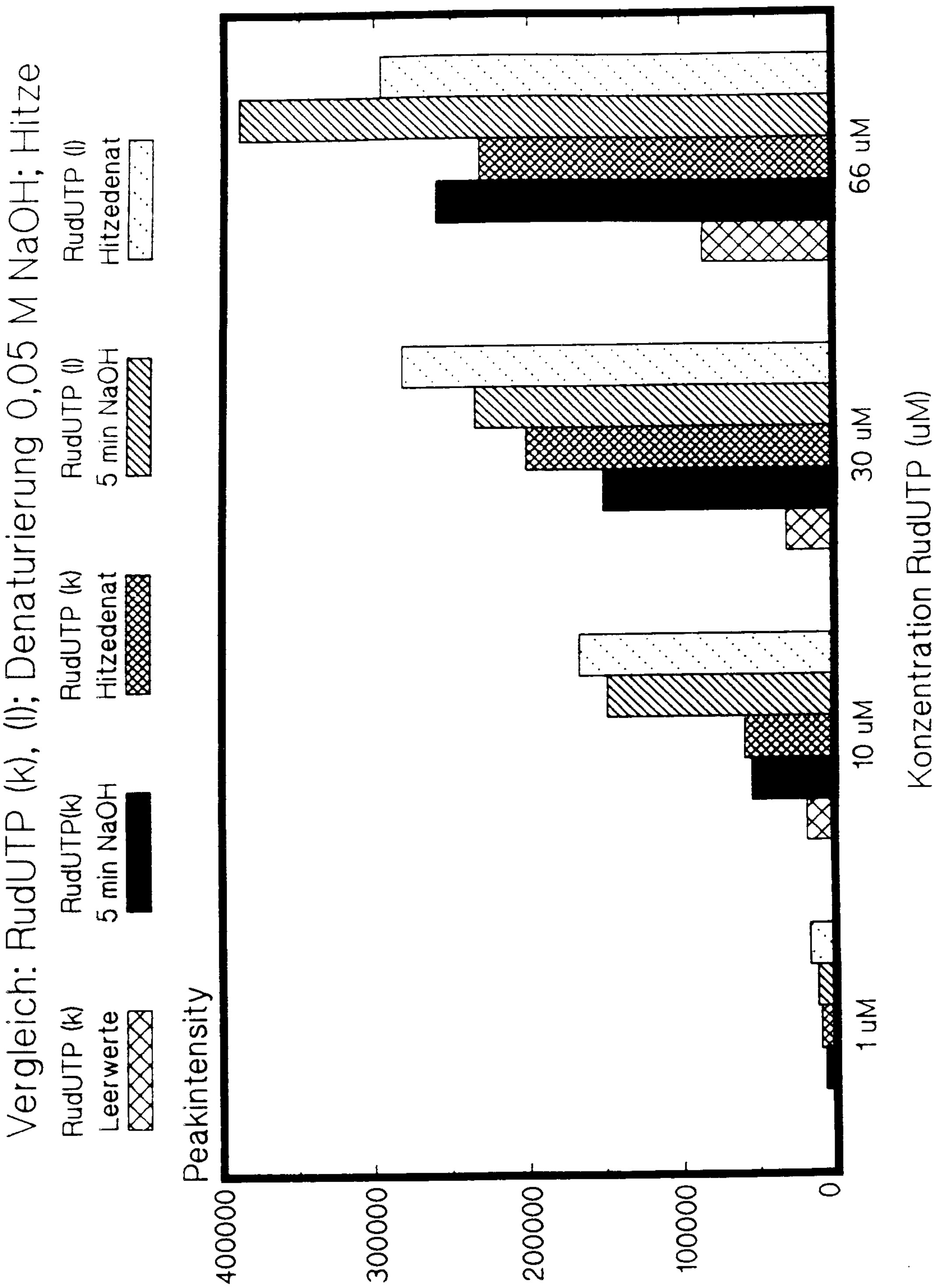
Vergleich: RudUTP (k), (l); Denaturierung 0,05 M NaOH; Hitze
RudUTP (k) Leerwerte
RudUTP(k) 5 min NaOH
RudUTP (k) Hitzedenat
RudUTP (l) 5 min NaOH
RudUTP (l) Hitzedenat
Peakintensity
400000
300000
200000
100000
0
1 uM
10 uM
30 uM
66 uM
Konzentration RudUTP (uM)

FIG 13

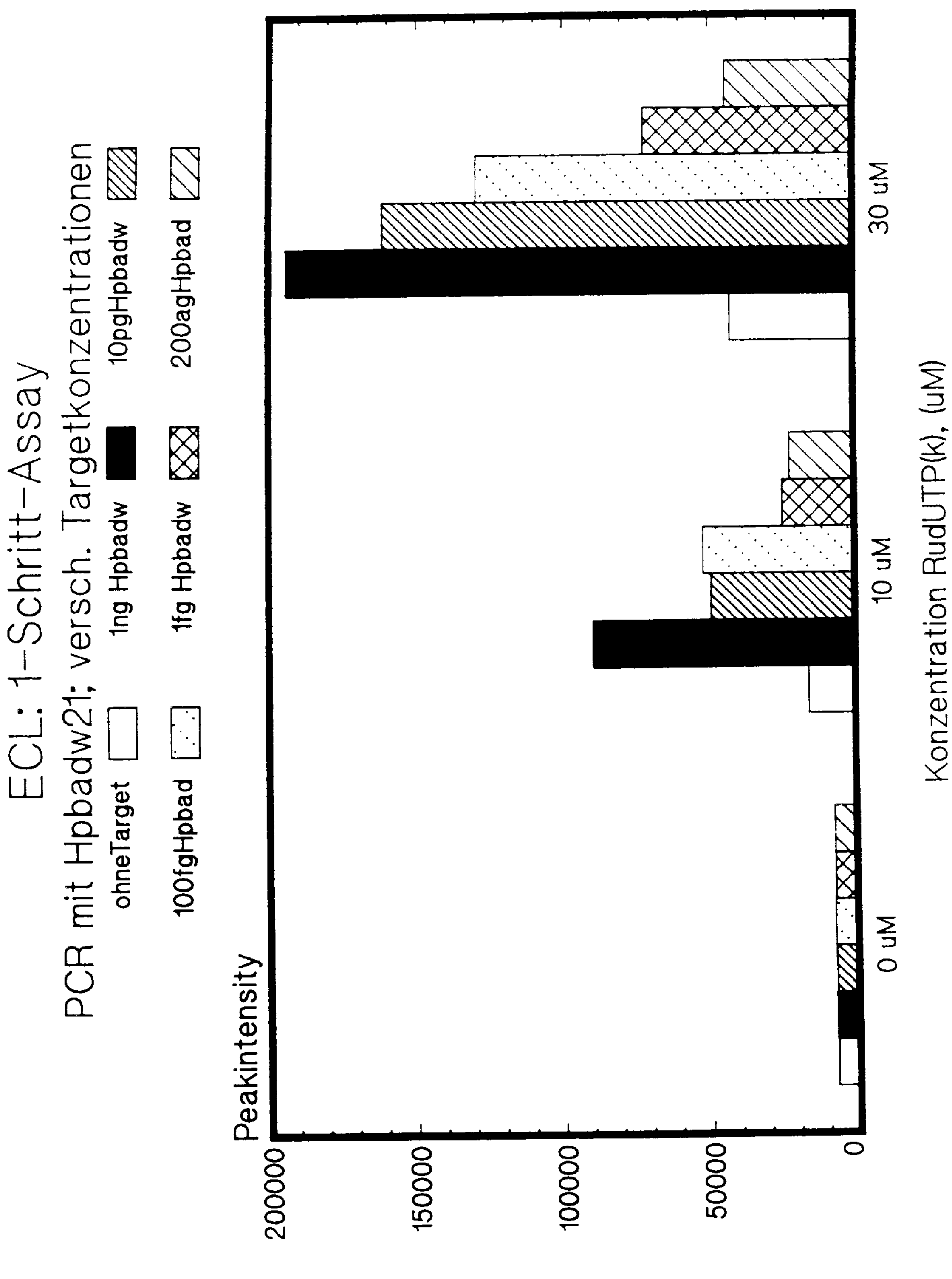
ECL: 1-Schritt-Assay
PCR mit Hpbadw21; versch. Targetkonzentrationen
ohneTarget
1ng Hpbadw
10pgHpbadw
100fgHpbad
1fg Hpbadw
200agHpbad
Peakintensity
200000
150000
100000
50000
0
0 uM
10 uM
30 uM
Konzentration RudUTP(k), (uM)

. 14

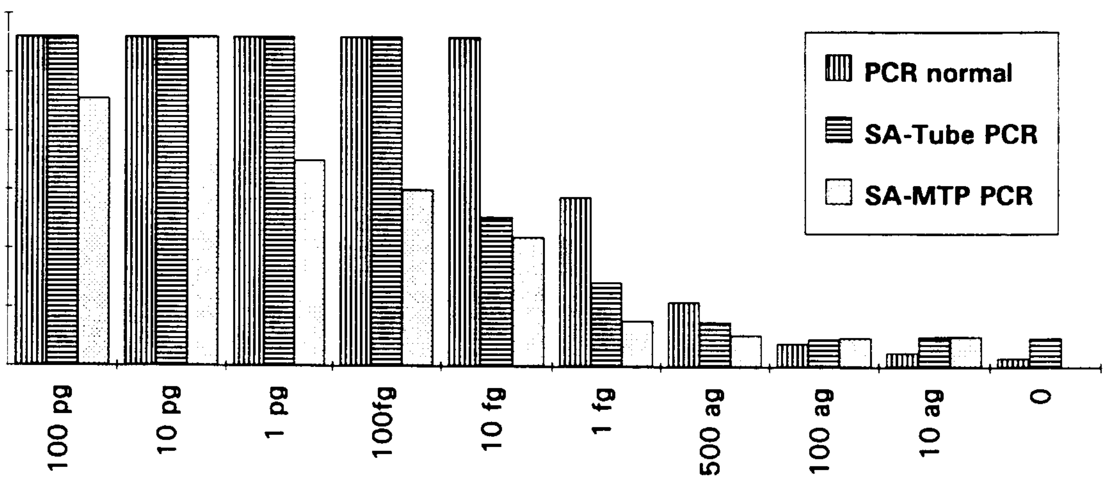
PCR normal
SA-Tube PCR
SA-MTP PCR
100 pg
10 pg
1 pg
100fg
10 fg
1 fg
500 ag
100 ag
10 ag
0
Hpbadw21 x HindIII

SEQUENZPROTOKOLL

(1) ALLGEMEINE INFORMATION:

(i) ANMELDER:
(A) NAME: Boehringer Mannheim GmbH
(B) STRASSE: Sandhoferstr.116
(C) ORT: Mannheim
(E) LAND: DE
(F) POSTLEITZAHL: 6800

(ii) ANMELDETITEL: Verfahren zur Immobilisierung von Nukleinsaeuren

(iii) ANZAHL DER SEQUENZEN: 11

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 18 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 1:

GGAGTGTGGA TTCGCACT 18

(2) INFORMATION ZU SEQ ID NO: 2:

(i) SEQUENZ CHARAKTERISTIKA:
(A) LÄNGE: 18 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 2:

TGAGATCTTC TGCGACGC 18

(2) INFORMATION ZU SEQ ID NO: 3:

(i) SEQUENZ CHARAKTERISTIKA:
(A) LÄNGE: 27 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 3:

AGCCTATAGA CCACCAAATG CCCCTAT 27

(2) INFORMATION ZU SEQ ID NO: 4:

(i) SEQUENZ CHARAKTERISTIKA:
(A) LÄNGE: 52 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 4:

AATTCTAATA CGACTCACTA TAGGGAGACG CGCTTTACCT GCTTCGGCGA TT 52

(2) INFORMATION ZU SEQ ID NO: 5:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 5:

GTAATCATCC GAAACCGCTC A 21

(2) INFORMATION ZU SEQ ID NO: 6:

(i) SEQUENZ CHARAKTERISTIKA:
(A) LÄNGE: 19 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 6:

CGTTTTACTT CTTGGACCG 19

(2) INFORMATION ZU SEQ ID NO: 7:

(i) SEQUENZ CHARAKTERISTIKA:
(A) LÄNGE: 19 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 7:

CGUUUUACUU CUUGGACCG 19

(2) INFORMATION ZU SEQ ID NO: 8:

(i) SEQUENZ CHARAKTERISTIKA:
(A) LÄNGE: 47 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 8:

AATTCTAATA CGACTCACTA TAGGGCCTGG CTTTAATTTT ACTGGTA 47

(2) INFORMATION ZU SEQ ID NO: 9:

(i) SEQUENZ CHARAKTERISTIKA:
(A) LÄNGE: 25 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 9:

ACAGGAGCAG ATGATACAGT ATTAG 25

(2) INFORMATION ZU SEQ ID NO: 10:

(i) SEQUENZ CHARAKTERISTIKA:
(A) LÄNGE: 31 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 10:

UGGAAGAAAU CUGUUGACUC AGAUUGGUUG C 31

(2) INFORMATION ZU SEQ ID NO: 11:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 11:

AGACCACCAA ATGCCCCTAT 20